# EUROPEAN PATENT SPECIFICATION

(11) **EP 1 676 831 B1**
(45) Date of publication and mention of the grant of the patent: **08.10.2008**
(21) Application number: 05024404.5
(22) Date of filing: 09.11.2005
(51) Int. Cl.: C07C 215/10, C07C 217/28, C11D 1/66, C11D 3/30, C09K 8/00, A01N 35/00, A61K 8/60

(54) **N,N-dialkylpolyhydroxyalkylamines**
N,N-Dialkylpolyhydroxyalkylamine
N,N-Dialkylpolyhydroxyalkylamines

(30) Priority: 11.11.2004 US 986749
(43) Date of publication of application: 05.07.2006
(73) Proprietor: Air Products and Chemicals, Inc., Allentown, PA 18195-1501 (US)
(72) Inventor: Meier, Ingrid Kristine, Asbury, NJ 08802 (US); Ford, Michael Edward, Trexlertown, PA 18087 (US); Goddard, Richard Joseph, Fogelsville, PA 18051 (US)
(74) Representative: Schwabe - Sandmair - Marx

(56) References cited:
- EP-A- 0 118 863
- US-A- 2 016 962
- US-A- 5 449 770
- US-A- 5 693 311
- US-A1- 2002 197 223
- WARWEL S ET AL: "SUFACTANTS FROM EPOXIDES AND POLYOLAMINES BASED ON DISACCHARIDES" TENSIDE, SURFACTANTS, DETERGENTS, CARL HANSER VERLAG, MUNCHEN, DE, vol. 40, no. 6, November 2003 (2003-11), pages 327-331, XP001185207 ISSN: 0932-3414

## Description

### FIELD OF THE INVENTION

This invention relates to surfactants. More particularly, it relates to N,N-dialkylpolyhydroxyalkylamines, their use as surfactants, and methods for making them.

### BACKGROUND OF THE INVENTION

The ability to reduce the surface tension of water is of great importance in the application of water-based formulations because decreased surface tension translates to enhanced substrate wetting during use. Examples of water-based compositions requiring good wetting include coatings, inks, adhesives, fountain solutions for lithographic printing, cleaning compositions, metalworking fluids, agricultural formulations, electronics cleaning and semiconductor processing compositions, personal care products, and formulations for textile processing and oilfield applications. Surface tension reduction in water-based systems is generally achieved through the addition of surfactants, resulting in enhanced surface coverage, fewer defects, and more uniform distribution. Equilibrium surface tension (EST) is important when the system is at rest, while dynamic surface tension (DST) provides a measure of the ability of a surfactant to reduce surface tension and provide wetting under high speed application conditions.

The importance of the ability of a surfactant to achieve low surface tension at low use levels, the ability to affect foaming performance, and the surfactant's ability to provide efficient emulsification and solubilization are all of considerable industrial importance, as is well-appreciated in the art. And, although equilibrium surface tension reduction efficiency is important for some applications, other applications may require both equilibrium and dynamic surface tension reduction.

The foaming characteristics of a surfactant are also important because they can help define applications for which the surfactant might be suitable. For example, foam can be desirable for applications such as ore flotation and cleaning. On the other hand, in coatings, graphic arts and adhesive applications, foam is undesirable because it can complicate application and lead to defect formation. Thus foaming characteristics are frequently an important performance parameter.

U.S. Pat. No. 5,449,770 discloses the preparation of N-alkylaminopolyols by reaction of N-alkylamines with reducing sugars in the presence of hydrogen and nickel catalysts. These compounds are useful as surfactants.

The wide variety of applications for which surfactants are used, and the resultant variation in performance requirements, results in a need for a correspondingly large number of surfactants adapted to these various performance demands, and a need for suitable methods for making them.

### SUMMARY OF THE INVENTION

In a first aspect, the invention relates to compounds according to formula (I): 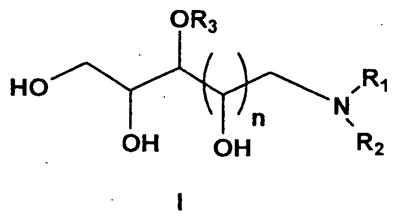 wherein n is an integer from 0 to 2; R₁ and R₂ are independently selected from the group consisting of C2 - C30 linear alkyl, cyclic alkyl, branched alkyl, alkenyl, aryl, aralkyl, alkaryl, alkoxyalkyl, and dialkylaminoalkyl; and R₃ is selected from the group consisting of hydrogen, α-D-glucopyranosyl, β-D-glucopyranosyl, and β-D-galactopyranosyl.

In a second aspect, the invention provides a method of making a compound according to formula (I) above. The method includes contacting an N-alkylpolyhydroxyalkylamine with a carbonyl equivalent compound selected from the group consisting of nitriles, ketones, aldehydes, acetals, and hemiacetals. The contacting is performed in the presence of hydrogen and a transition metal catalyst in a reaction solvent comprising at least 30 wt% of an organic solvent selected from the group consisting of methanol, isopropanol, tetrahydrofuran, ethylene glycol, propylene glycol, 1,2-dimethoxyethane, and mixtures of these.

In a third aspect, the invention provides a formulation containing between 0.1 and 99.9 wt% in total of one or more ingredients selected from the group consisting of surfactants and wetting agents other than according to formula (I) as shown above; solvents; alkali metal hydroxides; water-borne, water-dispersible, or water-soluble resins; flow agents; leveling agents; pigments; processing aids; defoamers; solubilizing agents; pesticides; plant growth modifying agents; water-soluble film-forming macromolecules; water-soluble alcohols, glycols, or polyols; water-soluble acids or salts thereof; tetramethylammonium hydroxide; emulsifying agents; alkanolamines; organic monoacids; biocides; chelants; detergent builders; detergent co-builders; dyes; fragrances; anti-redeposition aids; sunscreen agents; solubilizing agents; polymers; oligomers; functional cement additives; sodium chloride; sodium bromide; calcium chloride; calcium bromide; zinc chloride; zinc bromide; cesium formate; hydrochloric acid; hydrofluoric acid; acetic acid; formic acid; between 0.001 and 45 wt% of one or more compounds according to formula (I), and the balance water to 100 wt.%.

In a fourth aspect, the invention provides a fluid for drilling, completing, cementing, stimulating, fracturing, acidizing, or working over a subterranean gas or oil well, or for treating or enhancing the production of oil or gas from an oil or gas bearing formation. The fluid includes between 5 and 99.85 wt% in total of at least one of an organic liquid and water and further contains between 0.1 and 80 wt% in total of one or more ingredients selected from the group consisting of weighting agents, viscosifiers, dispersants, drilling mud base oils, emulsifiers, soluble salts, cements, proppants, mineral acids, organic acids, biocides, defoamers, demulsifiers, corrosion inhibitors, friction reducers, gas hydrate inhibitors, hydrogen sulfide removal or control additives, asphaltene control additives, paraffin control additives, and scale control additives, wherein the one or more ingredients does not include any component of a pre- or post-preparation synthesis reaction mixture for preparation of any of the one or more compounds according to formula (I) as shown above. The fluid also contains between 0.05 and 10 wt% of one or more compounds according to formula (I).

In a fifth aspect, the invention provides a method for drilling, completing, cementing, stimulating, fracturing, acidizing, working over, or treating a subterranean well. The method includes injecting into the well a fluid containing one or more compounds according to formula (I) as shown above.

In a sixth aspect, the invention provides a method for treating a produced stream of oil or gas from an oil and gas bearing formation. The improvement includes injecting into the produced stream a fluid comprising one or more compounds according to formula (I) as shown above.

In a seventh aspect, the invention provides a formulation including:
i) a first component consisting of one or more compounds according to formula (I) as shown above; and
ii) a second component consisting of one or more materials selected from the group consisting of nonvolatile organic and nonvolatile inorganic materials and mixtures of these. The second component does not including any component of a pre- or post-preparation synthesis reaction mixture for preparation of any of the one or more compounds according to formula (I), and the formulation is fluid at 25°C.

### DETAILED DESCRIPTION OF THE INVENTION

### N,N-Dialkylpolyhydroxyalkylamines

This invention relates to N,N-dialkylpolyhydroxyalkylamines according to formula (I), and methods of making and using them.

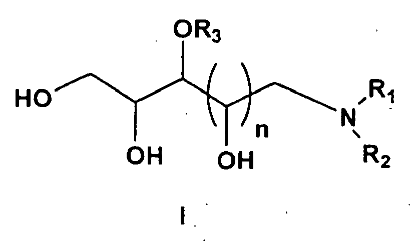

In formula (I), n is an integer from 0 to 2, and R₁ and R₂ are independently selected from the group consisting of C2 - C30 linear, cyclic, and branched alkyl, alkenyl, aryl, aralkyl, and alkaryl groups, or from the group consisting of C2 - C30 alkoxyalkyl and dialkylaminoalkyl groups. Typically, R₃ = H, for example when the compound according to formula (I) is derived from a monosaccharide. The compound of formula (I) may however instead be derived from a disaccharide, in which case R₃ is selected from the group consisting of α-D-glucopyranosyl, β-D-glucopyranosyl, and β-D-galactopyranosyl.

Although any of a variety of polyhydroxyalkyl groups may be incorporated in compounds according to the invention, they most typically will be derived from the open-chain forms of reducing sugars, for example glucose. Therefore, for simplicity of explanation, these compounds are exemplified herein as glucose derivatives such as may be obtained by the reaction of an N-(1-deoxyglucityl)alkylamine with an aldehyde or ketone, combined with a reduction employing a transition metal catalyst and hydrogen, as will be discussed below. Thus, exemplary glucose-derived compounds made according to the invention are of formula (I), wherein R₁ and R₂ are as defined above, n is 2, and R₃ is hydrogen.

The N-alkylpolyhydroxyalkylamine with which a carbonyl compound (or its equivalent) is reacted can be prepared by reductive amination of a polyhydroxyalkyl compound, such as a glucose or other suitable mono- or disaccharide, with the desired amine. One way of doing this is shown in U.S. Pat. No. 5,449,770, Example 1, where glucose reacts with R₁-NH₂ (where R₁ may for example be methyl) to give an N-alkylpolyhydroxyalkylamine according to formula (II), where n is 2 and R₃ is H.

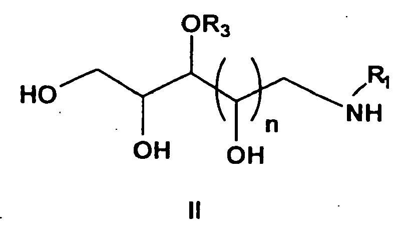

In general, the polyhydroxyalkyl groups of N-alkylpolyhydroxyalkylamines useful for making N,N-dialkylpolyhydroxyalkylamines according to the invention may be derived from any of the group of reducing sugars consisting of glucose, fructose, maltose, lactose, galactose, mannose, and xylose. Typically, the reducing sugar will be an aldose, although ketoses may also be used, and both monosaccharides and disaccharides may be used, with convenient sources of the latter including high dextrose corn syrup, high fructose corn syrup, and high maltose corn syrup. Other useful polyhydroxyalkyl groups may be derived from glyceraldehydes. In one embodiment, the polyhydroxyalkyl group is derived from glucose; i.e. the group is 1-deoxyglucityl.

The alkylamine with which the reducing sugar or other polyhydroxyalkyl group precursor is reacted may be represented by the formula R₁-NH₂. The group R₁ is as defined above. It may contain from 2 to 30 carbon atoms, preferably from 2 to about 18 carbon atoms, and more preferably from 2 to about 14 carbon atoms. Examples of suitable alkylamines include, but are not limited to, ethylamine, propylamine, isopropylamine, n-butylamine, isobutylamine, n-pentylamine, isopentylamine, cyclopentylamine, n-hexyl-amine, cyclohexylamine, n-heptylamine, n-octylamine, 2-ethylhexylamine, isooctylamine, n-decylamine, n-dodecylamine, 3-methoxypropylamine, 3-ethoxypropylamine, 3-n-propoxypropylamine, 3-isopropoxypropylamine, 3-n-hexyloxypropylamine, 3-isohexyloxy-propylamine, 3-[(2-ethyl)hexyloxy]propylamine, 3-isodecyloxypropylamine, 3-isotridecyloxypropylamine, 3-dodecyloxypropylamine, 3-isododecyloxypropylamine, 3-tetra-decyloxypropylamine, mixed octyloxy-decyloxypropylamines (Tomah PA-1214, available from Tomah Products, Inc. of Milton, WI), mixed tetradecyloxy-dodecyloxypropylamines (Tomah PA-1816), mixed dodecyloxy-tetradecyloxypropylamines (Tomah PA-1618), mixed dodecyloxy-pentadecyloxypropylamines (Tomah PA-19), mixed octadecyloxyhexadecyloxypropylamines (Tomah PA-2220), 3-dimethylaminopropylamine, 3-diethylaminopropylamine, 3-di-n-hexylpropylamine, stearylamine, and mixtures of amines derived from natural sources such as cocoalkylamine, oleylamine, and tallowamine. More preferred amines are ethylamine, butylamine, n-hexylamine, and n-octylamine. As used herein, the meaning of "alkylamine" as used in the terms "N-(1-deoxyglucityl)alkylamine" and "N-alkylpolyhydroxyalkylamine" is to be understood to include amines where the alkyl group is either substituted or unsubstituted, non-limiting examples of which are set forth in the foregoing part of this paragraph.

The carbonyl equivalent compound with which the intermediate N-alkyl-1-(deoxyglucityl)amine of formula (II) is reacted may be an aldehyde or a ketone, represented by formula (III).

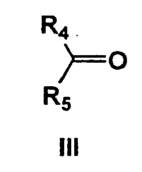

If the carbonyl equivalent compound is an aldehyde, R₄ is H and R₅ may be a linear, cyclic, or branched alkyl, alkoxylalkyl, dialkylaminoalkyl, alkenyl, aryl, aralkyl, or alkaryl group having from 1 to about 30 carbon atoms, preferably from about 1 to about 18 carbon atoms, and more preferably from about 1 to about 14 carbon atoms. Examples of suitable aldehydes include, but are not limited to, acetaldehyde, propionaldehyde, butyraldehyde, valeraldehyde, hexanal, cyclopentane carboxaldehyde, heptanal, cyclohexane carboxaldehyde, heptanal, octanal, nonanal, and decanal. Acetals and hemiacetals may also or instead be used in place of aldehydes, an nitriles may also be used, according to the invention. As used herein, the term "carbonyl equivalent compound" includes aldehydes, ketones, acetals, hemiacetals, and nitriles. For example, acetaldehyde dimethyl acetal and dimethylaminoacetaldehyde dimethyl acetal may be used.

If the carbonyl equivalent compound is a ketone, R₄ and R₅ may be independently selected from the group consisting of linear, cyclic, or branched alkyl, alkoxylalkyl, dialkylaminoalkyl, alkenyl, aryl, aralkyl, or alkaryl group having from 1 to about 30 carbon atoms, preferably from about 2 to about 18 carbon atoms, and more preferably from about 4 to about 14 carbon atoms. Examples of suitable ketones include, but are not limited to, acetone, methoxyacetone, 2-butanone, diethyl ketone, methyl isobutyl ketone, methyl isoamyl ketone, cyclopentanone, cyclohexanone, 2-heptanone, 2-octanone, 4-octanone, 2-nonanone, and 2-decanone.

Although compounds (I) of this invention may be made by reductive alkylation of an N-1-(deoxyglucityl)alkylamine with either an aldehyde or ketone, those made by reaction of the deoxyglucitylalkylamine with an aldehyde are preferred. More preferred are those derived from linear aldehydes such as acetaldehyde, butyraldehyde, valeraldehyde, or hexanal.

The preferred compounds (I) of the present invention are N-ethyl-N-pentyl-1-(deoxyglucityl)amine, N-ethyl-N-hexyl-1-(deoxyglucityl)amine, N-ethyl-N-octyl-1-(deoxyglucityl)amine, N,N-dibutyl-1-(deoxyglucityl)amine, N-butyl-N-pentyl-1 - (deoxyglucityl)amine, N-butyl-N-hexyl-1-(deoxyglucityl)amine, and N-butyl-N-octyl-1-(deoxyglucityl)amine. The more preferred compounds are N-ethyl-N-hexyl-1-(deoxyglucityl)amine, N,N-dibutyl-1-(deoxyglucityl)amine, N-butyl-N-pentyl-1-(deoxyglucityl)amine, and N-butyl-N-hexyl-1-(deoxyglucityl)amine.

### Preparation of Compounds of Formula (I)

In one exemplary preparative procedure, compounds according to the invention may be prepared by the reductive alkylation of an N-alkylpolyhydroxyalkylamine with an aldehyde or ketone, typically in the presence of a solvent, at a temperature sufficiently high so as to provide a convenient reaction rate and sufficiently low so as to prevent significant by-product formation. Functional equivalents of aldehydes and ketones, such as acetals/ketals (especially the dimethyl or diethyl acetals/ketals), hemiacetals/hemiketals, or nitriles may also be used. If acetals or ketals are used, it may be necessary to add a small amount of water to the reaction mixture, allow formation of the corresponding carbonyl compound. The reaction temperatures may be in the range from about 50°C to about 175°C, typically from about 50°C to about 150°C, and more typically from about 60°C to about 125°C. The optimum conditions will depend upon the reactor configuration, the solvents employed, and other variables. The N-alkylpolyhydroxyalkylamines may be prepared using procedures such as those described in US 5,449,770 to Shumate et al.

The linking of the N-alkylpolyhydroxyalkylamine with an aldehyde or ketone to form the corresponding compound (I) requires the presence of a catalyst and hydrogen. The catalyst is typically a metal chosen from the group consisting of iron, cobalt, nickel, ruthenium, rhodium, palladium, osmium, iridium or platinum. Typically, the catalyst is selected from the group consisting of ruthenium, rhodium, palladium, or platinum, and more typically is either palladium or platinum. To maximize productivity, the catalyst is typically dispersed on a support. Such a support may be organic, such as carbon, or inorganic. Examples of the latter class of supports include alumina, silica, titania, magnesia, zirconia, and aluminosilicates. The preferred support for the catalyst is carbon. The hydrogen pressure may be in the range from about 250 psig to 1500 psig, typically from about 500 psig to about 1250 psig, and more typically from about 750 psig to about 1100 psig.

A variety of solvents may be used in the reaction medium for the reductive alkylation of the N-alkylpolyhydroxyalkylamine. The inventors have found that the use of organic solvents for this purpose may result in suitably high reaction rates, reaction specificity, and ease of reaction mixture workup to isolate the desired product. Additionally, it has been found that reducing the concentration of water in the reaction medium may be beneficial to improving reaction rates and specificity. In many cases, however, reducing the amount of water in the reaction mixture to a sufficiently low level solely by increasing the concentration of reactants results in low solubility of starting materials and/or products, as well as excessively high reaction mixture viscosities. The latter condition may give rise to less-than-optimal heat transfer and/or hydrogen mass transfer, and thus is undesirable. The inventors have found that use of certain organic solvents in certain amounts allows such problems to be overcome or at least greatly diminished, while keeping the concentration of water sufficiently low that conversion rates and reaction specificity are good. Thus the term "reaction solvent", as that term is used herein, may mean one or more organic solvents, either with or without admixture with water.

In many cases, it is convenient to use, as starting material, an N-alkylpolyhydroxyalkylamine that has been generated *in situ* in the reaction equipment in which it will subsequently be coupled with an aldehyde or ketone to form the final product. Such an *in situ* preparation is typically performed by reductive reaction of a mono- or disaccharide with an amine, as noted herein above, and typically involves the use of significant water in the reaction medium to provide solubility for the saccharide and/or the product. This introduces a significant amount of water into the reaction mixture for the subsequent reductive alkylation reaction. However, especially when using aldehydes or ketones that contain four or more carbon atoms in their structures, solubility in an all-aqueous medium may be poor, resulting in slow reaction rates. Fortunately, the use of a significant proportion of organic solvent for the reasons described above typically helps to solubilize the starting material, thus providing an additional benefit.

Examples of suitable organic solvents and organic solvent mixtures include, but may not be limited to, methanol, ethanol, ethylene glycol, propylene glycol, tetrahydrofuran, isopropanol, 1,2-dimethoxyethane, and mixtures thereof. The most preferred organic solvent is methanol. The reductive alkylation to form N,N-dialkylpolyhydroxyalkylamines according to the invention may be performed in any of these solvents, or mixtures thereof with water, provided that the reaction solvent comprises at least 30 wt%, preferably at least 50 wt%, more preferably at least 70 wt%, and most preferably at least 80 wt% of an organic solvent or mixture thereof. The amount of water in the reaction solvent is thereby limited accordingly. In some cases, the reaction solvent is water-free, i.e. it contains only such small amounts of water as may have been adventitiously introduced with the organic solvent.

### Uses of Compounds of Formula (I) .

Compositions according to the invention may also include a variety of other ingredients adapted to complement the utility of compounds of formula (I) in a number of applications. The performance properties of such products may be optimized for a specific application by appropriate modification of the structure of the amine and the choice of the substituents R₁, R₂, and R₃. Such optimization is routine, and within the ability of the person of ordinary skill in the art in the particular application area. Thus manipulation of these variables yields compounds which may be useful as emulsifiers or detergents, wetting agents, foaming agents, defoamers, rheology modifiers or associative thickeners, dispersants, and the like. As such, these compounds may be useful in applications such as coatings, inks, adhesives, agricultural formulations, fountain solutions, photoresist strippers and developers, shampoos, and detergents and other cleaning compositions. The compounds may also find use in oil-field exploration, development, and production applications such as enhanced oil recovery, fracturing and stimulation processes, and drilling and cementing operations, and may also be useful in various wet-processing textile operations, such as dyeing of fibers and fiber scouring and kier boiling. The general formulation principles governing each of these applications are well known in the respective arts, and a detailed description of the numerous application areas and methods for incorporating the compounds of this invention into such formulations is not necessary to their effective incorporation therein. However, as an indication of the wide scope of possible uses for compounds according to the invention, exemplary but nonlimiting formulations are set forth below for a number of application areas.

The terms "water-based", "waterborne", "aqueous", or "aqueous medium", or "aqueous carrier" as used herein refer to systems in which the solvent or liquid dispersing medium comprises at least 50 wt% water, preferably at least 90 wt%, and more preferably at least 95 wt% water. The dispersing medium may consist essentially of water, i.e. it may have no added solvents, or it may also contain solvents.

In broad terms, compounds according to formula (I) may be used in a wide range of formulations that include a second component, such that the application of the second component benefits from the surface active properties provided by the formula (I) material. It is to be understood that, although components of a pre- or post-preparation synthesis reaction mixture for preparation of the compounds according to formula (I) may be present, these do not count as part of the second component for purposes of this invention. Such materials might for example include simple salts, solvents, catalysts, organic precursors, reagents, side products, and byproducts related to the preparation of the compound of formula (I), are not part of the second component. Typically, but not necessarily, the amount by weight of the second component in a formulation will be greater than that of the compound(s) of formula (I).

Formulations containing compounds according to formula (I) according to the invention are typically constructed so as to be fluid at 25°C. They are typically aqueous, but they need not be. The second component may consist of one or more materials selected from nonvolatile organic and nonvolatile inorganic materials, and mixtures of these. As used herein, the term "nonvolatile" means that the indicated material either cannot boil, or it boils at a temperature of at least 150°C at a pressure of 760 Torr. Thus, although typical low-boiling solvents may be included in the formulation, they do not constitute a part of the second component.

Typical non-limiting examples of nonvolatile materials are given in the exemplary formulations provided hereinafter. Formulations according to the invention may include ready-to-use formulations, or concentrates. Either of these may be further diluted in use. Thus the concentration of the one or more compounds of formula (I) in a composition according to the invention may vary over a wide range. Typically it will be between 0.001 and 45 wt% of the formulation, although in some cases the amount may be as low as 0.00001 wt%. In many cases compositions at the higher end of this concentration range will be diluted during or before use in the intended application, although this is not required in all applications.

By using compounds of formula (I), it is possible to reduce surface tension in a water-borne composition or an industrial process. Thus the invention provides aqueous compositions comprising such compounds, wherein the surfactant provides good wetting properties when used in a surfactant effective amount. For example, the amount of surfactant that is effective to provide enhanced wetting properties of a water-based, organic compound containing composition may range from 0.00001 to 5 wt%, preferably from 0.0001 to 3 wt%, and most preferably from 0.001 to 3 wt%, based on total weight of the formulation. The most favorable amount will vary from one application to another, depending upon the amount and type of other species present in the formulation that are capable of affecting foam properties and wetting performance, for example latex polymers.

A typical water-based coating formulation that includes the surfactants of the invention may include the following components in an aqueous medium, typically at 30 to 80% solids:

**Typical Aqueous-Based Coating Formulation**

| | |
|---|---|
| 0 to 50 wt% | Pigment Dispersant/Grind Resin |
| 0 to 80 wt% | Coloring Pigments/Extender Pigments/Anti-Corrosive Pigments/Other Pigment Types |
| 5 to 99.9 wt% | Water-Borne/Water-Dispersible/Water-Soluble Resins |
| 0 to 30 wt% | Slip Additives/Antimicrobials/Processing Aids/Defoamers |
| 0 to 50 wt% | Coalescing or Other Solvents |
| 0.01 to 10 wt% | Surfactant/Wetting/Flow and Leveling Agents, other than Compound of Formula (I) |
| 0.001 to 5 wt% | Compound(s) of Formula (I) |

A typical water-based ink composition that includes the surfactants of the invention may include the following components in an aqueous medium at a 20 to 60% solids content (i.e. not including the coalescing solvent):

**Typical Aqueous-Based Ink Composition**

| | |
|---|---|
| 1-50 wt% | Pigment |
| 0 to 50 wt% | Pigment Dispersant/Grind Resin |
| 0 to 50 wt% | Clay base in appropriate resin solution vehicle |
| 5 to 99.9 wt% | Water-borne/water-dispersible/water-soluble resins |
| 0 to 30 wt% | Coalescing Solvents |
| 0.01 to 10 wt% | Surfactant/Wetting Agents, other than Compound(s) of Formula (I) |
| 0.01 to 10 wt% | Processing Aids/Defoamers/Solubilizing Agents |
| 0.001 to 5 wt% | Compound(s) of Formula (I) |

A typical water-based agricultural composition that includes the surfactants of the invention may include the following components in an aqueous medium at 0.01 to 80% of the following ingredients:

**Typical Aqueous-Based Agricultural Composition**

| | |
|---|---|
| 0.1-50wt% | Pesticide or Plant Growth Modifying Agent |
| 0.01 to 10 wt% | Surfactants, other than Compound(s) of Formula (I) |
| 0 to 5 wt% | Dyes |
| 0 to 20 wt% | Thickeners/Stabilizers/Co-surfactants/Gel lnhibitors/Defoamers |
| 0 to 25 wt% | Antifreeze agent (e.g. ethylene glycol or propylene glycol) |
| 0.001 to 5 wt% | Compound(s) of Formula (I) |

A typical fountain solution composition for planographic printing that includes the surfactants of the invention may include the following components:

**Typical Fountain Solution for Planographic Printing**

| | |
|---|---|
| 0.05 to 10 wt% | Film forming, water soluble macromolecule |
| 1 to 25 wt% | C2-C12 Alcohol, glycol, or polyol (water soluble, or soluble due to use of a co-solvent) |
| 0.01 to 20 wt% | Water soluble organic acid, inorganic acid, or a salt of these |
| 30 to 98.9wt% | Water |
| 0.001 to 5 wt% | Compound(s) of Formula (I) |

A typical hard surface cleaner that includes the surfactants of the invention may include the following components:

**Typical Hard Surface Cleaner**

| | |
|---|---|
| O to 25 wt%* | Anionic surfactant |
| 0 to 25 wt% * | Cationic surfactant |
| 0 to 25 wt% * | Nonionic surfactant (e.g. alcohol alkoxylates, etc.) |
| 0 to 20 wt% | Chelating agent (EDTA, citrate, tartrate, etc.) |
| O to 20 wt%* | Solvent (Glycol ether, lower alcohols, etc.) |
| 0.001 to 25 wt% | Compound(s) of Formula (I) |
| 0 to 2 wt% | Dye, fragrance, preservative, etc. |
| 0 to 40 wt% * | Alkali metal hydroxide |
| Balance to 100 wt% | Water, and optionally other ingredients |

| | |
|---|---|
| *To total, in combination, between 0.1 and 99 wt%. | |

A typical water-based photoresist developer or electronic cleaning composition that includes the surfactants of the invention may include the following components:

**Typical Aqueous-Based Photoresist Developer Composition**

| | |
|---|---|
| 0.1 to 3 wt% | Tetramethylammonium hydroxide |
| 0 to 4 wt% | Phenolic resin |
| 92.5 to 99.9 wt% | Water |
| 0.001 to 5 wt% | Compound(s) of Formula (I) |

A typical metalworking fluid that includes the surfactants of the invention may include the following components:

**Typical Synthetic Metalworking Fluid Formulation**

| | |
|---|---|
| 2.5 to 10 wt% | Block copolymer or other emulsifying agent |
| 10 to 25 wt% | Alkanolamine |
| 2 to 10 wt% | Organic monoacid |
| 0 to 5 wt% | Organic diacid |
| 40 to 84.4 wt% | Water |
| 1 to 5 wt% | Biocide |
| 0.001 to 5 wt% | Compound(s) of Formula (I) |

Surfactants are also used in a wide variety of products in the areas of personal care and household and industrial cleaning. The surfactants of the present invention may be used in any of these formulations to provide one or more benefits, with the exact structure of the surfactant compound depending upon the specific performance features required for a particular application. Typical formulations used in these markets are described in Louis Ho Tan Tai's book, *Formulating Detergents and Personal Care Products: A Complete Guide to Product Development* (Champaign, IL: AOCS Press, 2000) as well as in other books, literature, product formularies, etc. familiar to those skilled in the art. A few representative example formulations are described here as illustrations. For example, a rinse aid for use in household automatic dishwashing or in industrial and institutional warewashing may have the ingredients described below.

**Typical Rinse Aid Formulation**

| | |
|---|---|
| Compound(s) of Formula (I) | 0.001 to 45 wt% |
| Nonionic surfactant other than a compound of Formula (I) (e.g. alkoxylated alcohol(s), alkoxylated block copolymers, etc.) | 0 to 45 wt% |
| Hydrotrope (e.g. sodium xylenesulfonate, sodium toluenesulfonate, anionic surfactant(s), amphoteric surfactant(s), etc.) | 0 to 10 wt% |
| Isopropyl alcohol or ethyl alcohol | 0 to 10 wt% |
| Chelant (e.g. citric acid, etc.) | 5 to 20 wt% |
| Water, and optionally other ingredients | Balance to 100 wt% |

**Typical Powdered Laundry Detergent Formulation**

| Material | Amount by Weight in Conventional Formulation | Amount by Weight in Concentrated Formulation |
|---|---|---|
| Compound(s) of Formula (I) | 0.001 to 5 wt% | 0.001 to 15 wt% |
| Detergent surfactant(s) (e.g. anionic surfactants, alcohol alkoxylates, etc.) | 0.1 to 30 wt% | 0.1 to 50 wt% |
| Builder/co-builder (zeolites, sodium carbonate, phosphates, etc.) | 25 to 50 wt% | 25 to 60 wt% |
| Bleach and bleach activator (perborates, etc.) | 0 to 25 wt% | 0 to 25 wt% |
| Other Additives (fragrance, enzymes, hydrotropes, etc.) | 0 to 7 wt% | 1 to 10 wt% |
| Fillers (sodium sulfate, etc.) | 5 to 35 wt% | 0 to 12 wt% |

**Typical Aqueous Liquid Laundry Detergent Formulation**

| Material | Amount by Weight in Conventional Formulation | Amount by Weight in Concentrated Formulation |
|---|---|---|
| Compound(s) of Formula (I) | 0.001 to 25 wt% | 0.001 to 30 wt% |
| Detergent surfactant(s) (e.g. anionic surfactants, alcohol alkoxylates, etc.) | 0 to 35 wt% | 0 to 65 wt% |
| Builder/co-builder (citrate, tartrate, etc.) | 3 to 30 wt% | 0 to 36 wt% |
| Other Additives (fragrances, dyes, etc.) | 0.1 to 5 wt% | 1 to 5 wt% |
| Water and other solvents (e.g. lower alcohols) | 5 to 75 wt% | 1 to 56 wt% |

**Typical Non-Aqueous Laundry Detergent Formulation**

| Material | Amount by Weight |
|---|---|
| Compound(s) of Formula (I) | 0.001 to 30 wt% |
| Detergent surfactant(s) (e.g. anionic surfactants, alcohol alkoxylates, amine oxides, etc.) | 0.1 to 42 wt% |
| Builder/co-builder (zeolites, sodium carbonate, phosphates, citrate or tartrate salts, etc.) | 25 to 60 wt% |
| Bleach and bleach activator (perborates, etc.) | 0 to 20 wt% |
| Anti-redeposition aids (sodium carboxymethylcellulose, etc.) | 0.5 to 5 wt% |
| Other Additives (fragrance, enzymes, etc.) | 0 to 5 wt% |
| Polyalkylene glycol | 0 to 50 wt% |

**Typical 2-Part Industrial and Institutional Laundry Formulation**

| | Amount by Weight of Material in Each Pack |
|---|---|
| **Pack A** | |
| Compound(s) of Formula (I) | 0.001 to 20 wt% |
| Detergent surfactant(s) (e.g. anionic surfactants, alcohol alkoxylates, etc.) | 0 to 20 wt% |
| Antiredeposition aids (sodium carboxymethyl-cellulose, etc.) | 0.01 to 2 wt% |
| Water, and optionally other ingredients | Balance to 100 wt% |
| **Pack B** | |
| Sodium silicate | 5 to 10 wt% |
| Sodium metasilicate | 0 to 30 wt% |
| Tetrapotassium pyrophosphate | 0 to 10 wt% |
| potassium hydroxide | 0 to 35 wt% |
| potassium carbonate | 0 to 15 wt% |
| Water, and optionally other ingredients | Balance to 100 wt% |
| **Mix Ratio Pack A:Pack B** | 1:2 to 1:4 |

**Typical Shampoo or Liquid Body Wash Formulation**

| Material | Amount by Weight |
|---|---|
| Compound(s) of Formula (I) | 0.001 to 5 wt% |
| Anionic surfactant(s) (e.g. sodium or ammonium lauryl sulfate, sodium or ammonium lauryl sulfate, etc.) | 0.1 to 30 wt% |
| Amphoteric cosurfactant(s) (e.g. cocoamidopropyl betaine, etc.) | 0 to 20 wt% |
| Nonionic surfactant other than a compound of Formula (I) (e.g. alcohol alkoxylates, sorbitan esters, alkyl glucosides, etc.) | 0 to 20 wt% |
| Cationic polymers (e.g. polyquaternium, etc.) | 0 to 5 wt% |
| Other Additives (fragrance, dyes, oils, opacifiers, preservatives, chelants, hydrotropes, etc.) | 0 to 15 wt% |
| Polymeric thickeners (e.g. polyacrylate, etc.) | 0 to 2 wt% |
| Conditioning oils (e.g. sunflower oil, petrolatum, etc.) | 0 to 10 wt% |
| Citric acid | 0 to 2 wt% |
| Ammonium chloride or sodium chloride | 0 to 3 wt% |
| Humectants (e.g. propylene glycol, glycerin, etc.) | 0 to 15 wt% |
| Glycol distearate | 0 to 5 wt% |
| Cocoamide (i.e. cocoamide MEA, cocoamide MIPA, PEG-5 cocoamide, etc.) | 0 to 10 wt% |
| Dimethicone | 0 to 5 wt% |
| Behenyl alcohol | 0 to 5 wt% |
| Water, and optionally other ingredients | Balance to 100 wt% |

**Typical Hair Conditioner Formulation**

| Material | Amount by Weight |
|---|---|
| Compound(s) of Formula (I) | 0.001 to 10 wt% |
| Nonionic surfactant other than a compound of Formula (I), and/or fatty alcohol(s) (e.g. stearyl alcohol, etc.) | 0.1 to 10 wt% |
| Cationic surfactant(s) (e.g. cetrimonium chloride, etc.) | 0 to 10 wt% |
| Anionic surfactants (e.g. TEA-dodecylbenzenesulfonate, etc.) | 0 to 5 wt% |
| Silicones (e.g. dimethicone, dimethiconal, etc.) | 0 to 5 wt% |
| Cationic polymers (e.g. polyquaternium, etc.) | 0 to 10 wt% |
| Other Additives (fragrance, dyes, oils, opacifiers, preservatives, chelants, hydrotropes, etc.) | 0 to 10 wt% |
| Thickening polymers (e.g. hydroxyethylcellulose, polyacrylates, etc.) | 0 to 5 wt% |
| Potassium, ammonium or sodium chloride | 0 to 5 wt% |
| Humectant (e.g. propylene glycol, etc.) | 0 to 5 wt% |
| Panthenol | 0 to 2 wt% |
| Water, and optionally other ingredients | Balance to 100 wt% |

**Typical Aqueous Sunscreen Formulation**

| Material | Amount by Weight |
|---|---|
| Compound(s) of Formula (I) | 0.001 to 30 wt% |
| Polyethylene glycol (e.g. PEG-8, etc.) | 0 to 30 wt% |
| Active sunscreen agents (e.g. octyl methoxycinnamate, azobenzone, homosalate, octyl salicylate, oxybenzone, octocrylene, butyl methoxydibenzoylmethane, octyl triazone, etc.) | 1 to 30 wt% |
| Esters and emollients (e.g. dimethicone, methylparaben, propylparaben, polysorbates, etc.) | 0 to 20 wt% |
| Thickening polymers (e.g. acrylates/C10-30 alkyl acrylate crosspolymer, PVP/hexadecene copolymer, etc.) | 0 to 20 wt% |
| Other Additives (fragrance, dyes, oils, opacifiers, preservatives, chelants, etc.) | 0 to 15 wt% |
| Solvent/hydrotropes (e.g. propylene glycol, benzyl alcohol, dicapryl ether, etc.) | 0 to 20 wt% |
| Triethanolamine | 0 to 5 wt% |
| Water, and optionally other ingredients | Balance to 100 wt% |

### Cement Admixture Formulations

Cement admixtures may be of any of several types, including superplasticizing, plasticizing, accelerating, set retarding, air entraining, water-resisting, corrosion inhibiting, and other types. Such admixtures are used to control the workability, settling and end properties (strength, impermeability, durability and frost/deicing salt resistance, etc.) of cementitious products like concretes, mortars, etc. The admixtures are usually provided as aqueous solutions and they can be added to the cementitious system at some point during its formulation. Surfactants of this invention may provide wetting, foam control, flow and leveling, water reduction, corrosion inhibition, high ionic strength tolerance and compatibility, and other benefits when used in such systems.

**Exemplary Cement Admixture Ingredients**

| Material | Amount by Weight Relative to Cement Weight |
|---|---|
| Compound(s) of Formula (I) | 0.001 to 5 wt% |
| Solubilizing agents (solvent, hydrotropes, amines, etc.) * | 0 to 10 wt% |
| Polymers and/or oligomers (e.g. lignosulfonates, sulfonated melamine formaldehyde condensates, polycarboxylates, styrene-maleic anhydride oligomers, copolymers and their derivatives, etc.)* | 0 to 5 wt% |
| Functional Additives (defoamers, air entraining or detraining agents, pH control additives, corrosion inhibitors, set retarders, accelerators, preservatives, etc.) * | 0 to 5 wt% |
| Water | 40 to 75% |

| | |
|---|---|
| *To total, in combination, between 0.1 and 20 wt%. | |

### Oil and Gas Field Formulations

Surfactants of this invention, used alone or as a component in formulations, may provide surface tension reduction, foam control, and improved wetting in a variety of applications within the Oil and Gas industry. These may include, for example, formulations for the following uses.

In drilling applications, the surfactants may be used in formulations for dispersion of clays and drill cuttings, ROP (rate of penetration) enhancement, emulsification and deemulsification, surface wetting and surface tension reduction, shale stabilization, and enhancement of hydration or dissolution of solid additives.

In cementing, stimulation and workover applications, uses may include formulations for spacers, cement dispersion, controlled air content in cements, cement retardation, fracturing fluids, stimulation of coal bed methane, surface or interfacial tension reduction, oil/water wetting, and cleaning fluids.

In oil and gas production, uses may include rig wash formulations, defoaming of crude, water flooding/injection, defoaming for acid gas sweetening, oil/water separation, enhanced oil recovery, and inhibition or dispersion of asphaltenes, hydrates, scale and waxes.

Exemplary fluids for drilling, completing, cementing, stimulating, fracturing, acidizing, working over, or other treating of subterranean wells, or for enhancing production from an oil- or gas-bearing formation or treating the produced oil or gas, typically include from 0.05 to 10 wt% of a surfactant of this invention in a fluid containing water and/or an organic liquid, which typically constitutes from 5 to 99.85 wt% of the fluid. The organic liquid is typically a petroleum product, although it need not be, and may for example include crude oil or any of the drilling mud base oils described below. If water is included, it may be from a freshwater, sea water, or brine source, or it may be provided by inclusion of an aqueous mineral acid such as hydrochloric acid, hydrofluoric acid, sulfuric acid, etc. Fluids for such applications usually also include between 0.1 and 80 wt% in total of one or more ingredients selected from weighting agents, viscosifiers, dispersants, drilling mud base oils, emulsifiers, soluble salts, cements, proppants, mineral acids, organic acids, biocides, defoamers, demulsifiers, corrosion inhibitors, friction reducers, gas hydrate inhibitors, hydrogen sulfide removal or control additives, asphaltene control additives, paraffin control additives, and scale control additives. A variety of specific materials are known in the art for performing these functions. Suitable nonlimiting examples of some of these materials follow, and others will be apparent to those of skill in the art.
*Weighting agents:* barium sulfate, hematite, and ilmenite.
*Viscosifiers:* clays (e.g. bentonite, attapulgite), water-soluble polymers (e.g. xanthan gum, guar, polysaccharides, modified polysaccharides), organophilic clays, and oil-soluble polymers.
*Dispersants:* lignosulfonates, naphthalene sulfonates, sulfonated melamine formaldehyde resins.
*Drilling mud base oils:* diesel, mineral oil, olefinic oils, paraffinic oils, and esters.
*Emulsifiers:* fatty acids, fatty amides, anionic surfactants, and nonionic alkoxylated surfactants.
*Soluble salts (e. g. for specific gravity adjustment, shale stabilization, or osmotic pressure control):* NaCl, NaBr, KCI, KBr, CaCl₂, CaBr₂, ZnCl₂, ZnBr₂, sodium formate, potassium formate, and cesium formate.
*Cements*
*Other Surfactants:* cationic surfactants, amphoteric surfactants, alkyl glucosides, phosphate esters, and fluorosurfactants.
*Proppants:* ceramics, sintered bauxite, sand, and resin-coated sand.
*Organic Acids:* formic acid, acetic acid, citric acid.
*Mineral acids:* hydrochloric acid and hydrofluoric acid.

The foregoing classes of materials may find application, when used in combination with the surfactants of this invention, in a variety of oilfield applications. Depending upon the exact application and desired effect, compositions may be injected into a well or added to the stream of oil or gas produced by the well, all according to methods well known in the art.

Typical applications, and the ingredients commonly (although not necessarily) used in making formulations for these purposes, are shown immediately below. Other ingredients may also be present. It will be understood that each of these formulations will also contain a surfactant according to the invention.
*Water-based drilling muds:* weighting agents, viscosifiers, and dispersants.
*Oil-based drilling muds:* base oil, emulsifier, and viscosifier.
*Completion fluids:* soluble salts for specific gravity adjustment.
*Cement Formulations:* the cements themselves, in combination with dispersants.
Spacers: weighting agents and surfactants.
*Acidizing fluids:* surfactants and one or both of mineral acids and organic acids.
*Fracturing fluids:* viscosifiers, proppants, and surfactants.

Fluids for stimulating or enhancing production from a gas or oil bearing formation, may contain ingredients similar to those found in fracturing fluids, except for proppants. Finally, fluids for treating oil or gas produced in the above ways may include one or more of biocides, defoamers, demulsifiers, corrosion inhibitors, friction reducers, gas hydrate inhibitors, hydrogen sulfide removal or control additives, asphaltene control additives, paraffin control additives, and scale control additives.

Compounds according to formula (I) may have utility in preventing or slowing the formation of gas hydrates in petroleum-bearing formations or during transport of crude petroleums, where lower hydrocarbons such as methane, ethane, propane, n-butane, and isobutane are commonly found. Water is also typically present in such formations and, under conditions of elevated pressure and reduced temperature, mixtures of the water and lower hydrocarbons tend to form clathrate hydrates. Such hydrates are water crystals that have formed a cage structure around a guest molecule such as the lower hydrocarbon. For example, at a pressure of about 1 MPa, ethane can form gas hydrates with water at temperatures below 4°C; at a pressure of 3 MPa, it can form gas hydrates with water at temperatures below 14°C. Temperatures and pressures such as these are commonly encountered for many environments in which natural gas and crude petroleum are produced and transported. The resulting hydrates frequently cause pipeline blockages due to growth and agglomeration of crystals inside pipes, conduits, valves, and other equipment, resulting in reduced flow and even equipment damage.

Compounds according to formula (I) may be used to reduce the nucleation, growth, and/or agglomeration of gas hydrates by including them in petroleum streams, thereby minimizing unscheduled shutdowns, maintenance and repair. The amount of compound of formula (I) used for such an application may vary over a wide range, and may depend *inter alia* upon the relative proportions of the various lower hydrocarbons present in the crude petroleum, the temperature and pressure conditions to which the petroleum will be exposed, and the amount of water present. An appropriate amount for any given situation can easily be determined by routine experimentation, but typically the amount will be at least about 0.05 wt% relative to the amount of water present, more typically at least about 0.1 wt%, and most typically at least about 0.3 wt%. While there need be no upper limit to the amount of compound of formula (I) used, it may be most economical to limit it to at most about 5 wt% relative to water and typically at most 2 wt% relative to water. In one embodiment of the invention, a compound of formula (I) in which n is 2, R₃ is H, R₁ is n-butyl, and R₂ is n-pentyl is used as a gas hydrate inhibitor.

As will be appreciated in light of the foregoing discussion, the compounds of this invention may find utility in a wide variety of applications. The present invention is further illustrated by the following examples, which are presented for purposes of demonstrating, but not limiting, the methods and compositions of this invention.

### EXAMPLES

### Examples 1 - 12c

Examples 1 - 12c illustrate a preferred process of this invention, reacting a 1-deoxy-1-(alkylamino)-D-glucitol with an aldehyde or ketone in the presence of a catalyst at elevated temperature and pressure of hydrogen. This transformation is illustrated by the following schematic equations for reaction of a 1-deoxy-1-(alkylamino)-D-glucitol with an aldehyde (where R₄ = H) or a ketone: 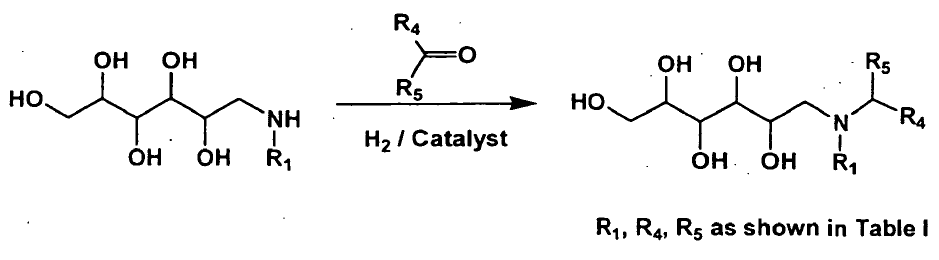 The reactions were performed in general according to a procedure similar to the following illustrative example, where N-butyl-N-pentyl-1-(deoxyglucityl)amine was prepared.

A 300 mL Autoclave Engineers stainless steel reactor was charged with 47.4g (0.20 mole) 1-deoxy-1-(butylamino)-D-glucitol, 17.9g (0.21 mole; 1.05 equivalent) of valeraldehyde, 1.28g (dry weight basis) 5% palladium on carbon, and 100g of methanol. The reactor was closed, purged with nitrogen and hydrogen, and pressurized to ca 600 psig with hydrogen. The mixture was heated with stirring (1000 rpm) to 100°C and pressurized with hydrogen to 1000 psig. The reaction was maintained at this temperature; pressure was maintained at 1000 psig via regulated hydrogen feed. After 10 hr, the mixture was cooled to 50°C, and the product removed from the reactor by filtration through an internal 0.5µm sintered metal element. Upon cooling, the product precipitated as a white ― cream colored solid. After trimethylsilylation, analysis of the product by gas chromatography (GC) and GC-MS (gas chromatography-mass spectrometry) indicated that it consisted of >95% N-butyl-N-pentyl-1-(deoxyglucityl)amine, and minor amounts of byproducts. ¹³C NMR (CDCl₃): 13.67, 13.71 (2C, CH₃); 20.02, 20.20 (1C, CH₂); 22.05, 22.22 (1C, CH₂); 25.78 (1C, CH₂); 28.28 (1C, CH₂); 29.23 (1C, CH₂); 53.44, 53.73 (2C, NCH₂CH₂-); 56.48 (1C, NCH₂CHOH-); 63.91 (1C, CH₂OH); 69.77, 70.89, 71.20 (3C, CHOH); 73.24 (1C, HOCH₂CHOH-) ppm.

Additional N,N-dialkyl-1-(deoxyglucityl)amines were prepared and characterized using procedures similar to that above. Details of the reactants used in these preparations are shown in Table 1 as sample Nos. 1 - 12c. Reaction conditions used for the runs in Table 1 are shown in Table 2.

**Table 1. Dialkyl Glucamine Preparation in Methanol^{a}**

| No. | R₁ | R₄ | R₅ | Sample Composition^{c} | | |
|---|---|---|---|---|---|---|
| | | | | Starting Glucamine | Dialkylglucamine | Other |
| 1 | C₂H₅ | H | n-C₅H₁₁ | <1 | 97 | 1 (aldol)^{d}; 2 unkn^{f} |
| 1b | n-C₄H₉ | | n-C₅H₁₁ | <0.5 | 99 | <0.5 |
| 2 | n-C₄H₉ | CH₃ | CH₃ | <1 | 99 | ND^{e} |
| 3 | n-C₄H₉ | H | n-C₃H₇ | 2 | 95 | 3 (aldol) |
| 4 | n-C₄H₉ | H | n-C₄H₉ | 2 | 98 | ND |
| 5 | n-C₆H₁₃ | H | CH₃ | ND | 99 | 1 (aldol) |
| 6 | n-C₆H₁₃ | H | n-C₃H₇ | ND | 97 | 3 (aldol) |
| 7 | n-C₆H₁₃ | H | n-C₅H₁₁ | 18 | 81 | 1 (aldol) |
| 8 | n-C₈H₁₇ | H | CH₃ | ND | 95 | 5 (aldol) |
| 9 | n-C₈H₁₇ | H | n-C₃H₇ | 2 | 97 | 3 (aldol) |
| 10. | (2-ethylhexyl)-O-(CH₂)₃- | H | CH₃ | 3 | 95 | 2 (aldol) |
| 11 | C₈H₁₇-O-(CH₂)₃- and C₁₀H₂₁-O-(CH₂)₃- | H | CH₃ | 3 | 95 | 2 (aldol) |
| 12 | i-C₁₀H₂₁-O-(CH₂)₃- | H | CH₃ | 7 | 92 | <1 (aldol) |
| 12a | n-C₁₂H₂₅ | H | CH₃ | <1 | 99 | 1 (aldol) |
| 12b | n-C₁₀H₂₁ | H | CH₃ | ND | 96 | 2 (aldol 2 unkn |
| 12c | Cocoalkyl^{g} | H | CH₃ | ND | 100 | ND |

| | | | | | | |
|---|---|---|---|---|---|---|
| Notes to table: a) Samples were isolated typically by precipitation and filtration. Compositions refer to the solid products. Complete mass balances were not done. b) Ratio by weight. c) GC analyses are area percent. d) Aldol reaction of aldehyde with itself, sometimes with reductive dehydration of the aldol product, followed by reductive alkylation of the alkylglucamine. e) None detected. f) Not identified. g) Mixture of C8, C10, C12, C14, C16, and C18 species, centered on C12. | | | | | | |

**Table 2: Experimental-Details for Table 1**

| No. | T (°C) | P (psig) | [reactants]^{a} | Catalyst (Wt%)^{b} | t (hr) | Scale |
|---|---|---|---|---|---|---|
| 1 | 100 | 1000 | 51 | 2.0 | 10 | 1.5 Gallon |
| 1b | 100 | 1000 | 41 | 2.0 | 10 | 300 mL |
| 2 | 100 | 1000 | 33 | 1.6 | 10 | 100 mL |
| 3 | 100 | 1000 | 13 | 2.0 | 10 | 300 mL |
| 4 | 100 | 1000 | 14 | 2.0 | 10 | 300 mL |
| 5 | 100 | 1000 | 34 | 2.0 | 10 | 100 mL |
| 6 | 50 | 1000 | 36 | 2.0 | 10 | 100 mL |
| 7 | 100 | 1000 | 28 | 2.0 | 10 | 100 mL |
| 8 | 100 | 1000 | 36 | 2.0 | 10 | 100 mL |
| 9 | 100 | 1000 | 38 | 2.0 | 10 | 100 mL |
| 10 | 100 | 1000 | 33 | 2.0 | 10 | 100 mL |
| 11 | 100 | 1000 | 33 | 2.0 | 10 | 100 mL |
| 12 | 100 | 1000 | 34 | 2.0 | 10 | 100 mL |
| 12a | 100 | 1000 | 29 | 2.0 | 10 | 100 mL |
| 12b | 100 | 1000 | 30 | 2.0 | 10 | 100 mL |
| 12c | 100 | 1000 | 25 | 0.6 | 10 | 300 mL |

| | | | | | | |
|---|---|---|---|---|---|---|
| a) Wt% starting materials (alkylglucamine and aldehyde) in reaction mixture. b) Catalyst loading (dry weight basis), based on total reactants (alkylglucamine and aldehyde). | | | | | | |

### Examples A-N Reaction Medium Effects

A series of preparations, labeled A-N, were made of N-butyl-N-pentylglucamine. The procedures used were as follows, with the results tabulated below in Table 3.

### Examples B-K, M, and N

Butylglucamine (11.85g; 0.05 mole) and 5% Pd/C (0.32g, dry weight basis; 2 wt%, based on total organic feed) were charged to a 100 mL Parr reactor bowl, followed by pentanal (4.5g; 0.0523 mole; 1.036 eq.). If appropriate, solvent components were mixed prior to addition to the reactor. In all cases, a total of 30g of (mixed) solvent was added to the reactor. Subsequently, the reactor was assembled, pressurized with nitrogen to 1000 psig, and vented to ca 5 ― 10 psig. After two more nitrogen pressurization ― vent cycles, the reactor was pressurized to 1000 psig with hydrogen, and the contents stirred at 1000 rpm until the pressure stabilized. (Typically, an initial drop of ca 40 - 80 psig was noted owing to solution of hydrogen in the reaction mixture.) The stirrer was turned off, and the reactor was leak checked. The reactor was vented to ca 5 ― 10 psig, the hydrogen pressurization - vent cycle was repeated two more times, and the reactor was then pressurized to 600 psig. The stirrer was then turned on to 1000 rpm again , and the reactor heated to 100°C. At 100°C, the pressure was adjusted to 1000 psig and maintained at that level via regulated hydrogen feed for the course of the reaction. The reaction was maintained at 100°C/1000 psig for 5 hr, after which time heating was stopped and the reaction mixture was cooled to ambient. The following morning, the reactor was opened. Reaction mixtures where the desired product was essentially fully soluble were filtered to remove catalyst, the filtrate was evaporated to dryness, and the product was trimethylsilylated and analyzed by GC. Reaction mixtures where the desired product was partially soluble were mixed with additional methanol to dissolve the product, and then filtered and treated as above.

### Example A

The above procedure was followed, but using 40 g of water and a reaction time of 10hr. (See Note d, Table 3.)

### Example L

The procedure of Example 6, DE 4307163 was followed on the 0.445X scale, with substitution of 6.885g (0.08008 mole; 1.067 eq.; the same ratio as used in that patent) of pentanal for aqueous formaldehyde. After being weighed in a pycnometer, the Raney® 2800 catalyst was washed with deionized water three times and transferred to the reactor with the butylglucamine - pentanal - water solution. The sequence of nitrogen and hydrogen pressurization - vent cycles was analogous to that immediately above, with the exception that the reactor was leak checked at 500 psig, pressurized to 250 psig prior to heating, and the final pressure adjusted to 440 psig at 100°C. The reaction was maintained at 100°C/440 psig for 10 hours, after which heating was stopped and the reaction cooled to ambient. The following morning, the semi-solid product was dissolved in added methanol, filtered, and the filtrate was evaporated to dryness. After trimethylsilylation, the product was analyzed by GC.

Table 3 shows the results of the above Examples A-N.

**Table 3. Effect of Reaction Medium on N-Butyl-N-pentylglucamine Preparation**

| | | | | Analysis^{c} | | |
|---|---|---|---|---|---|---|
| No. | Medium^{a} | Product Solubility | Conv(%)^{b} | Starting Glucamine | Dialkylglucamine | Others |
| A | Water^{d} | Partial | 88 | 12 | 88 | ND^{e} |
| B | Water^{d} | Partial | 98 | 2 | 98 | ND |
| C | Methanol/H₂O | Partial | 97 | 3 | 97 | ND |
| D | Methanol/H₂O (1:1) | Full | 100 | ND | 100 | ND |
| E | Methanol/H₂O (4:1) | Full | 100 | ND | 100 | ND |
| F | Methanol | Full | 100 | ND | 100 | ND |
| | | | | | | |
| G | THF/H₂O (1:1) | Full | 94 | 3 | 94 | 3^{f} |
| H | n-Butanol/H₂O (1:1) | Full | 98 | 2 | 98 | ND |
| I | n-Pentanol/H₂O (1:1) | Full | 97 | 3 | 97 | ND |
| J | Propylene Glycol/H₂O (1:1) | Full | 98 | 2 | 98 | ND |
| K | 1,2-Dimethoxyethane/H₂O (1:1) | Full | 98 | 2 | 98 | ND |
| | | | | | | |
| L | Water | Partial | 59 | 41 | 59 | <1^{g} |
| M | Water | Partial | 52 | 48 | 52 | <1^{g} |
| N | Water | Full | 60 | 40 | 60 | ND |

| | | | | | | |
|---|---|---|---|---|---|---|
| Notes to table: a) Weight ratios of organic solvent:water. b) Conversion based on starting alkylglucamine. c) GC analyses by area percent; the entire reaction mixture was evaporated, and a portion analyzed. d) Run A used 40g of water, run B used 30g of water. e) Not detected. f) Unknown material. g) Aldol reaction side products present at 0.2 ― 0.3%. | | | | | | |

The results shown in Table 3 indicate that minimization of water concentration in the reaction mixture favored rapid and selective formation of the desired dialkylglucamine. For example, runs A and B both used only water as the reaction medium, but run A used 40 mL of water and run B used only 30 mL, i.e. run B had a lower molar concentration of water in the reaction mixture, and exhibited more complete conversion of the butylglucamine starting material to desired product, despite a reaction time of only 5 hours for run B vs. 10 hours for run A. The effects of water concentration on conversion were distinct from effects of reactant concentration, as seen by comparing runs A and F. In both cases, a similar volume of reaction medium was used (40g = 40 mL of water in run A, 30g = 38 mL of methanol in run F), but considerably better conversion was obtained in run F, where no water was included in preparing the reaction medium. In particular, reaction media containing methanol showed particularly good conversions and selectivities, as seen by comparing run D with runs G-K.

### Examples 13-25

Equilibrium surface tensions obtained using N,N-dialkylpolyhydroxyalkylamines, and comparison runs using the corresponding N-alkylglucamine precursors, were determined using a Kruss K-12 tensiometer with a platinum Wilhelmy plate, maintaining the temperature at 25 ± 1°C by means of a constant temperature circulating bath. The results, reported in Table 4, are averages of 10 measurements over a 10-minute period, and have a standard deviation of less than 0.01 dyne/cm.

**Table 4 - Equilibrium Surfactant Data for N,N-Dialkyl-(1-deoxyglucityl)amines**

| EXAMPLE | SURFACTANT | EST (0.1 WT%, mN/m) | EST (1.0wt%, mN/m) |
|---|---|---|---|
| Comparative Example 13 | N-Butylglucamine | NA | ≥ 50 |
| Comparative Example 14 | N-Hexylglucamine | 60 | 52 |
| Comparative Example 15 | N-Octylglucamine | 35 | 35 |
| 16 | N-Ethyl-N-hexylglucamine | 43 | 32 |
| 17 | N-Ethyl-N-octylglucamine | 31 | 31 |
| 18 | N-Ethyl-N-(2-ethylhexyloxypropyl)glucamine | 30 | 30 |
| 19 | N-Ethyl-N-(octyl/decyloxypropyl)glucamine | 30 | 30 |
| 20 | N-Ethyl-N-(isodecyloxypropyl)glucamine | 29 | 29 |
| 21 | N,N-Dibutylglucamine | 55 | 37 |
| 22 | N-Butyl-N-pentylglucamine | 43 | 29 |
| 23 | N-Butyl-N-hexylglucamine | 32 | 32 |
| 24 | N-Butyl-N-octylglucamine | 32 | 32 |
| 25 | N,N-Dihexylglucamine | 33 | 33 |

Some of the practical benefits of a low equilibrium surface tension values are that less surfactant is required to reduce the surface tension of a formulation (enhancing its wetting properties) and less surfactant will be needed to stabilize emulsions. For the examples in Table 4, surfactants of the invention demonstrated low equilibrium surface tension values, which will enhance the ability of formulations containing them to wet out a given surface.

### Examples 26-38

An additional benefit that the surfactants of the invention offer is the reduction of dynamic surface tension. Solutions of the surfactants of the invention were prepared in distilled and deionized water. Their dynamic surface tensions were measured using the maximum bubble pressure method, and the resulting data are provided in Table 5. The maximum bubble pressure method of measuring surface tension is described in Langmuir 1986, 2, 428-432. These data provide information about the performance of a surfactant at conditions close to equilibrium (0.1 bubbles/sec) through high surface creation rates or dynamic conditions (10 bubbles/sec). In a practical sense, high surface creation rates refer to rapid processes such as a spray or roller-applied coating, a high speed printing operation, or the rapid application of an agricultural product or a cleaner.

**Table 5 - Dynamic Surface Tension Data for N,N-Dialkyl-1-(deoxyglucityl)amines**

| EXAMPLE | SURFACTANT | DST (0.1 WT%, mN/m) 0.1 b/s | DST (0.1 WT%, mN/m) 10b/s | DST (0.5 WT%, mN/m) 0.1 b/s | DST (0.5 WT%, mN/m) 10b/s |
|---|---|---|---|---|---|
| Comparative Example 26 | N-Butylglucamine | 71 | 72 | 70 | 71 |
| Comparative Example 27 | N-Hexylglucamine | 68 | 70 | 55 | 57 |
| Comparative Example 28 | N-Octylglucamine | 54 | 65 | 48 | 62 |
| 29 | N-Ethyl-N-hexylglucamine | 51 | 58 | 36 | 43 |
| 30 | N-Ethyl-N-octylglucamine | 35 | 41 | NA | NA |
| 31 | N-Ethyl-N-(2-ethylhexyloxypropyl)glucamine | 31 | 42 | NA | NA |
| 32 | N-Ethyl-N-(octyl/decyloxypropyl)glucamine | 29 | 45 | NA | NA |
| 33 | N-Ethyl-N-(isodecyloxypropyl) glucamine | 27 | 44 | NA | NA |
| 34 | N,N-Dibutylglucamine | 54 | 56 | 40 | 43 |
| 35 | N-Butyl-N-pentylglucamine | 45 | 49 | 32 | 35 |
| 36 | N-Butyl-N-hexylglucamine | 35 | 39 | NA | NA |
| 37 | N-Butyl-N-octylglucamine | 33 | 58 | NA | NA |
| 38 | N, N-Dihexylglucamine | 31 | 49 | NA | NA |

The data of Table 5 indicate that a wide range of dynamic surface tension reduction levels is possible with this family of molecules, providing different surfactants for strong (Examples 30, 31 and 36 at low bubble rates), and moderate to low (Example 29, 34 and 35) surface tension reduction of an aqueous solution or a formulation. Depending upon the mode of application of a formulation, the solubility of the surfactant in the formulation and the substrate to be wetted (brush application of an industrial coating, spray application of an industrial cleaner, roller application of an adhesive), surfactants that provide such a range of dynamic surface tension reduction may find significant commercial utility.

### Examples 39-51

The foaming characteristics of surfactants according to formula (I) were determined using a slight modification of the Ross-Miles foam test (Am. *Soc. For Testing Materials,* Method D1173-53, Philadelphia, PA, **1953)** for solutions of 0.1 wt% surfactant in water. Foam data for surfactants of the invention as well as the N-alkylglucamine precursors (comparative examples) are shown in Table 6.

**Table 6 - Foam Stability Data for N,N-Dialkyl-1-(deoxyglucityl)amines**

| EXAMPLE | SURFACTANT | Ross Miles Initial Foam Height (cm) | Ross Miles Final Foam Height After 5 min (cm) |
|---|---|---|---|
| Comparative Example 39 | N-Butylglucamine | 2.5 | 0.3 |
| Comparative Example 40 | N-Hexylglucamine | 2.0 | 1.0 |
| Comparative Example 41 | N-Octylglucamine | 2.0 | 0.3 |
| 42 | N-Ethyl-N-hexylglucamine | 0.9 | 0 |
| 43 | N-Ethyl-N-octylglucamine | 9.4 | 4.8 |
| 44 | N-Ethyl-N-(2-ethylhexyloxypropyl)glucamine | 1.2 | 0 |
| 45 | N-Ethyl-N-(octyl/decyloxypropyl)glucamine | 10.2 | 8.0 |
| 46 | N-Ethyl-N-(isodecyloxypropyl)glucamine | 10.4 | 8.2 |
| 47 | N,N-Dibutylglucamine | 1.0 | 0 |
| 48 | N-Butyl-N-pentylglucamine | 1.1 | 0 |
| 49 | N-Butyl-N-hexylglucamine | 1.1 | 0 |
| 50 | N-Butyl-N-octylglucamine | 2.0 | 0.4 |
| 51 | N,N-Dihexylglucamine | 1.1 | 0 |

For the N-alkylglucamine precursors to the invention, little change in initial foam height was observed with an increase in hydrophobe length (Comparative Examples 39, 40, and 41). The surfactants of the invention showed an increase in initial foam height and foam stability with an increase in alkyl chain length (compare Example 43 with 42, 45 and 46 with 44, and 50 with 49). These data demonstrate that a range of foam performance may be obtained, depending upon the alkyl group attached to the amine. While applications such as coatings, inks, and adhesives require low foam or foam that dissipates quickly, other applications such as cleaning or ore floatation require a controlled amount of foam to be present and to persist. Therefore, the surfactants of the invention will likely be of use in a wide range of applications where manipulation of foaming performance is important.

### Examples 52-53 - Gas Hydrate Inhibition

### Example 52 -Control Sample.

In this example, 100 mL of deionized water is charged to a 500-cc stainless steel autoclave equipped with a turbine agitator. The system is pressurized to 6.5 MPa with methane, and the pressure is maintained by the gas supply. The autoclave temperature is reduced at a rate of approximately 5°C per hour, to a temperature of 15°C. The temperature is then further reduced slowly in controlled fashion at a rate of 1°C per hour. Significant hydrate formation occurs at 9°C.

### Example 53 ―Use of Inhibitors.

The operation is the same as in Example 52, but 0.5 wt% (with respect to water) of a compound according to formula (I) is added to the autoclave prior to pressurization with methane. Significantly less nucleation, growth, and/or agglomeration of gas hydrates is observed than in the control experiment.

The invention provides novel surfactants with properties that make suitable for use in a wide range of industrial and commercial applications. Such applications include water-based coatings, inks, adhesives, agricultural formulations, aqueous and non-aqueous cleaning compositions, personal care applications, and formulations for textile processing and oilfield applications.

## Claims

1. A method of making a compound according to formula (I): 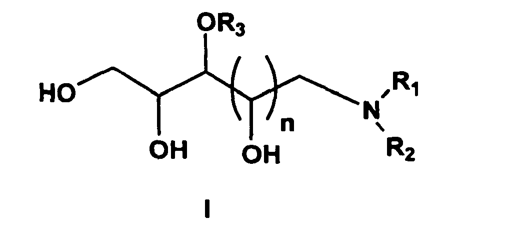 wherein n is an integer from 0 to 2; R₁ and R₂ are independently selected from the group consisting of C2 - C30 linear alkyl, cyclic alkyl, branched alkyl, alkenyl, aryl, aralkyl, alkaryl, alkoxyalkyl, and dialkylaminoalkyl; and R₃ is selected from the group consisting of hydrogen, α-D-glucopyranosyl, β-D-glucopyranosyl, and β-D-galactopyranosyl;
the method comprising contacting an N-alkylpolyhydroxyalkylamine with a carbonyl equivalent compound selected from the group consisting of nitriles, ketones, aldehydes, acetals, and hemiacetals, said contacting performed in the presence of hydrogen and a transition metal catalyst in a reaction solvent comprising at least 30 wt% of an organic solvent selected from the group consisting of methanol, isopropanol, tetrahydrofuran, ethylene glycol, propylene glycol, 1,2-dimethoxyethane, and mixtures of these.

2. The method of claim 1, wherein the organic solvent constitutes at least 50 wt% of the reaction solvent, preferably at least 70 wt% of the reaction solvent.

3. The method of claim 1, wherein the organic solvent comprises methanol, and preferably is methanol and the reaction mixture is water-free.

4. The method of claim 1, wherein the carbonyl equivalent compound is an aldehyde.

5. The method of claim 1, wherein n is 2 and R₃ is H.

6. The method of claim 7, wherein R₁ is ethyl and R₂ is selected from the group consisting of n-pentyl, n-hexyl, and n-octyl, preferably n-hexyl; or R₁ is n-butyl and R₂ is selected from the group consisting of n-butyl, n-pentyl, n-hexyl, and n-octyl, preferably n-butyl, n-pentyl, and n-hexyl; and wherein most preferably R₁ is n-butyl and R₂ is n-pentyl.

7. The method of claim 6, wherein the reaction solvent comprises at least 70 wt% of methanol.

8. The method of claim 1, wherein the catalyst is selected from the group consisting of iron, cobalt, nickel, ruthenium, rhodium, palladium, osmium, iridium and platinum, preferably ruthenium, rhodium, palladium, and platinum, more preferably palladium or platinum.

9. The method of claim 1, wherein the catalyst is supported on carbon.

10. The method of claim 1, wherein the contacting is performed at a temperature between about 50°C and about 175°C.

11. A formulation comprising between 0.1 and 99.9 wt% in total of one or more ingredients selected from the group consisting of surfactants and wetting agents other than according to formula (I); solvents; alkali metal hydroxides; water-borne, water-dispersible, or water-soluble resins; flow agents; leveling agents; pigments; processing aids; defoamers; solubilizing agents; pesticides; plant growth modifying agents; water-soluble film-forming macromolecules; water-soluble alcohols, glycols, or polyols; water-soluble acids or salts thereof; tetramethylammonium hydroxide; emulsifying agents; alkanolamines; organic monoacids; biocides; chelants; detergent builders; detergent co-builders; dyes; fragrances; anti-redeposition aids; sunscreen agents; solubilizing agents; polymers; oligomers; functional cement additives; sodium chloride; sodium bromide; calcium chloride; calcium bromide; zinc chloride; zinc bromide; cesium formate; hydrochloric acid; hydrofluoric acid; acetic acid; and formic acid;
between 0.001 and 45 wt% of one or more compounds according to formula (I): 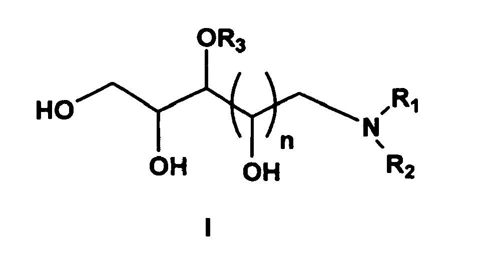 wherein n is an integer from 0 to 2; R₁ and R₂ are independently selected from the group consisting of C2 - C30 linear alkyl, cyclic alkyl, branched alkyl, alkenyl, aryl, aralkyl, alkaryl, alkoxyalkyl, and dialkylaminoalkyl; and R₃ is selected from the group consisting of hydrogen, α-D-glucopyranosyl, β-D-glucopyranosyl, and β-D-galactopyranosyl, as defined in one of claims 1 to 10; and the balance water to 100 wt%;
wherein said one or more ingredients do not include any component of a pre- or post-preparation synthesis reaction mixture for preparation of any of the one or more compounds according to formula (I).

12. The formulation of claim 11, wherein the formulation is a hard surface cleaning formulation comprising water and between 0.1 and 99 wt% in total of one or more ingredients selected from the group consisting of anionic surfactants, cationic surfactants, nonionic surfactants other than according to formula (I), solvents, and alkali metal hydroxides, the formulation comprising between 0.001 and 25 wt% of one or more compounds of formula (I).

13. The formulation of claim 11, wherein the formulation is a coating formulation comprising between 5 and 99.9 wt% of a water-borne, water-dispersible, or water-soluble resin, and between 0.01 and 10 wt% in total of one or more other additives selected from the group consisting of surfactants, wetting agents, and flow and leveling agents, other than according to formula (I), the formulation comprising between 0.001 and 5 wt% of one or more compounds of formula (I).

14. The formulation of claim 11, wherein the formulation is an ink formulation comprising between 1 and 50 wt% of a pigment, between 5 and 99.9 wt% of a water-borne, water-dispersible, or water-soluble resin, between 0.01 and 10 wt% of a surfactant or wetting agent other than according to formula (I), and between 0.01 and 10 wt% in total of one or more other additives selected from the group consisting of processing aids, defoamers, and solubilizing agents, the formulation comprising between 0.001 and 5 wt% of one or more compounds of formula (I).

15. The formulation of claim 11, wherein the formulation is an agricultural formulation comprising between 0.1 and 50 wt% of a pesticide or plant growth modifying agent and between 0.01 and 10 wt% of a surfactant or wetting agent other than according to formula (I), the formulation comprising between 0.001 and 5 wt% of one or more compounds of formula (I).

16. The formulation of claim 11, wherein the formulation is a fountain solution formulation for planographic printing comprising between 0.05 and 10 wt% of a water-soluble, film forming macromolecule, between 1 and 25 wt% of a water-soluble alcohol, glycol, or polyol, between 0.01 and 20 wt% of a water-soluble acid or its salt, and between 30 and 98.9 wt% of water, the formulation comprising between 0.001 and 5 wt% of one or more compounds of formula (I).

17. The formulation of claim 11, wherein the formulation is a photoresist developer formulation comprising between 0.1 and 3 wt% of tetramethylammonium hydroxide and between 92.5 and 99.9 wt% of water, the formulation comprising between 0.001 and 5 wt% of one or more compounds of formula (I).

18. The formulation of claim 11, wherein the formulation is a synthetic metalworking fluid formulation comprising between 2.5 and 10 wt% of an emulsifying agent, between 10 and 25 wt% of an alkanolamine, between 2 and 10 wt% of an organic monoacid, between 1 and 5 wt% of a biocide, and between 40 and 84.4 wt% of water, the formulation comprising between 0.001 and 5 wt% of one or more compounds of formula (I).

19. The formulation of claim 11, wherein the formulation is a rinse aid formulation comprising water and between 5 and 20 wt% of a chelant, the formulation comprising between 0.001 and 45 wt% of one or more compounds of formula (I).

20. The formulation of claim 11, wherein the formulation is a powdered laundry detergent formulation comprising between 0.1 and 50 wt% of one or more detergent surfactants and between 25 and 60 wt% of a builder or co-builder, the formulation comprising between 0.001 and 15 wt% of one or more compounds of formula (I).

21. The formulation of claim 11, wherein the formulation is an aqueous liquid laundry detergent formulation comprising between 0.1 and 65 wt% of one or more detergent surfactants, between 3 and 36 wt% of a builder or co-builder, between 0.1 and 5 wt% in total of one or more other additives selected from the group consisting of fragrances and dyes, and between 1 and 75 wt% in total of one or more other additives selected from the group consisting of water and other solvents, the formulation comprising between 0.001 and 30 wt% of one or more compounds of formula (I).

22. The formulation of claim 11, wherein the formulation is a non-aqueous laundry detergent formulation comprising between 0.1 and 42 wt% of one or more detergent surfactants, between 25 and 60 wt% of a builder or co-builder, and between 0.5 and 5 wt% of an anti-redeposition aid, the formulation comprising between 0.001 and 30 wt% of one or more compounds of formula (I).

23. The formulation of claim 11, wherein the formulation is an industrial and institutional laundry detergent formulation comprising water and between 0.01 and 2 wt% of an anti-redeposition aid, the formulation comprising between 0.001 and 20 wt% of one or more compounds of formula (I).

24. The formulation of claim 11, wherein the formulation is a shampoo or liquid body wash formulation comprising water and between 0.1 and 30 wt% of an anionic surfactant, the formulation comprising between 0.001 and 5 wt% of one or more compounds of formula (I).

25. The formulation of claim 11, wherein the formulation is a hair conditioner formulation comprising water and between 0.1 and 10 wt% of a nonionic surfactant other than according to formula (I), the formulation comprising between 0.001 and 10 wt% of one or more compounds of formula (I).

26. The formulation of claim 11, wherein the formulation is an aqueous sunscreen formulation comprising water and between 1 and 30 wt% of a sunscreen agent, the formulation comprising between 0.001 and 30 wt% of one or more compounds of formula (I).

27. The formulation of claim 11, wherein the formulation is a cement admixture formulation comprising between 40 and 75 wt% of water and between 0.1 and 20 wt% in total of one or more solubilizing agents, polymers, oligomers, or functional additives, the formulation comprising between 0.001 and 5 wt% of one or more compounds of formula (I).

28. A fluid for drilling, completing, cementing, stimulating, fracturing, acidizing, or working over a subterranean gas or oil well, or for treating or enhancing the production of oil or gas from an oil or gas bearing formation; the fluid comprising between 5 and 99.85 wt% in total of at least one of an organic liquid and water and further comprising between 0.1 and 80 wt% in total of one or more ingredients selected from the group consisting of weighting agents, viscosifiers, dispersants, drilling mud base oils, emulsifiers, soluble salts, cements, proppants, mineral acids, organic acids, biocides, defoamers, demulsifiers, corrosion inhibitors, friction reducers, gas hydrate inhibitors, hydrogen sulfide removal or control additives, asphaltene control additives, paraffin control additives, and scale control additives;
and between 0.05 and 10 wt% of one or more compounds according to formula (I): 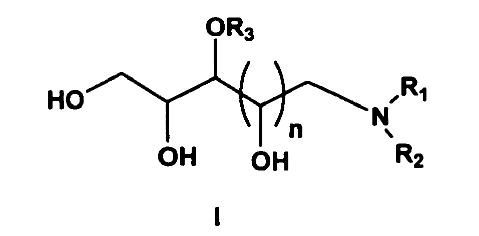 wherein n is an integer from 0 to 2; R₁ and R₂ are independently selected from the group consisting of C2 - C30 linear alkyl, cyclic alkyl, branched alkyl, alkenyl, aryl, aralkyl, alkaryl, alkoxyalkyl, and dialkylaminoalkyl; and R₃ is selected from the group consisting of hydrogen, α-D-glucopyranosyl, β-D-glucopyranosyl, and β-D-galactopyranosyl; as defined in one of claims 1 to 10; and
wherein said one or more ingredients do not include any component of a pre- or post-preparation synthesis reaction mixture for preparation of any of the one or more compounds according to formula (I).

29. A method for drilling, completing, cementing, stimulating, fracturing, acidizing, working over, or treating a subterranean well, said method including the step of injecting into the well a fluid comprising one or more compounds according to formula (I): 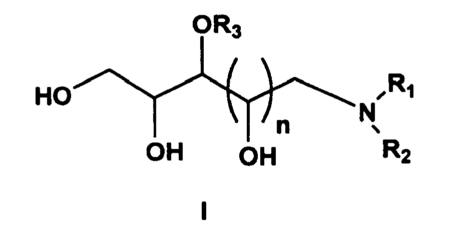 wherein n is an integer from 0 to 2; R₁ and R₂ are independently selected from the group consisting of C2 - C30 linear alkyl, cyclic alkyl, branched alkyl, alkenyl, aryl, aralkyl, alkaryl, alkoxyalkyl, and dialkylaminoalkyl; and R₃ is selected from the group consisting of hydrogen, α-D-glucopyranosyl, β-D-glucopyranosyl, and β-D-galactopyranosyl, as defined in one of claims 1 to 10.

30. The method of claim 29, wherein the method comprises treating the well to inhibit gas hydrate formation.

31. A method for treating a produced stream of oil or gas from an oil and gas bearing formation, said method comprising the step of injecting into the produced stream a fluid comprising one or more compounds according to formula (I): 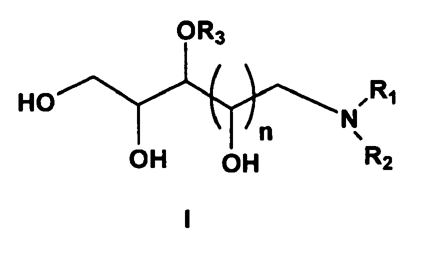 wherein n is an integer from 0 to 2; R₁ and R₂ are independently selected from the group consisting of C2 - C30 linear alkyl, cyclic alkyl, branched alkyl, alkenyl, aryl, aralkyl, alkaryl, alkoxyalkyl, and dialkylaminoalkyl; and R₃ is selected from the group consisting of hydrogen, α-D-glucopyranosyl, β-D-glucopyranosyl, and β-D-galactopyranosyl, as defined in one of claims 1 to 10.

32. The method of claim 31, wherein the method comprises treating the produced stream to inhibit gas hydrate formation.

33. A formulation comprising:
i) a first component consisting of one or more compounds according to formula (I): 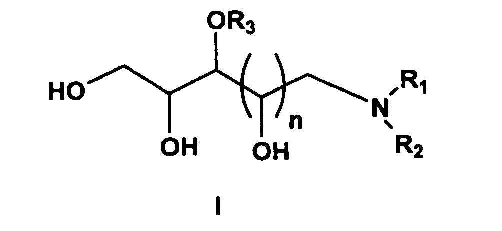 wherein n is an integer from 0 to 2; R₁ and R₂ are independently selected from the group consisting of C2 - C30 linear alkyl, cyclic alkyl, branched alkyl, alkenyl, aryl, aralkyl, alkaryl, alkoxyalkyl, and dialkylaminoalkyl; and R₃ is selected from the group consisting of hydrogen, α-D-glucopyranosyl, β-D-glucopyranosyl, and β-D-galactopyranosyl; as defined in one of claims 1 to 10 and
ii) a second component consisting of one or more materials selected from the group consisting of nonvolatile organic and nonvolatile inorganic materials and mixtures of these, said second component not including any component of a pre- or post-preparation synthesis reaction mixture for preparation of any of the one or more compounds according to formula (I);
wherein the formulation is fluid at 25°C.

34. The formulation of claim 33, wherein the second component is present in a greater amount by weight than the first component.

35. The formulation of claim 34, wherein the formulation comprises an aqueous carrier.

36. The formulation of claim 33, wherein the second component consists of one or more materials selected from the group consisting of surfactants or wetting agents other than according to formula (I); solvents; alkali metal hydroxides; water-borne, water-dispersible, or water-soluble resins; flow agents; leveling agents; pigments; processing aids; defoamers; solubilizing agents; pesticides; plant growth modifying agents; water-soluble film-forming macromolecules; water-soluble alcohols, glycols, or polyols; water-soluble acids or salts thereof; tetramethylammonium hydroxide; emulsifying agents; alkanolamines; organic monoacids; biocides; chelants; detergent builders; detergent co-builders; dyes; fragrances; anti-redeposition aids; sunscreen agents; solubilizing agents; polymers; oligomers; and functional cement additives.

37. The formulation of claim 36, wherein R₁ is ethyl and R₂ is selected from the group consisting of n-pentyl, n-hexyl, and n-octyl, preferably n-hexyl; or R₁ is n-butyl and R₂ is selected from the group consisting of n-butyl, n-pentyl, n-hexyl, and n-octyl, preferably n-butyl, n-pentyl, and n-hexyl; and wherein most preferably R₁ is n-butyl and R₂ is n-pentyl.

38. The use of a compound of formula (I) as defined in of claims 1 to 10 in a formulation selected from the group consisting of a hard surface cleaning formulation, a coating formulation, an ink formulation, an agricultural formulation, a fountain solution formulation, a photoresist developer formulation, a synthetic metalworking fluid formulation, a rinse aid formulation, a powdered laundry detergent formulation, an aqueous liquid laundry detergent formulation, a non-aqueous laundry detergent formulation, an industrial and institutional laundry detergent formulation, a shampoo or liquid body wash formulation, a hair conditioner formulation, an aqueous sunscreen formulation and a cement admixture formulation.

39. A compound of the formula (I): 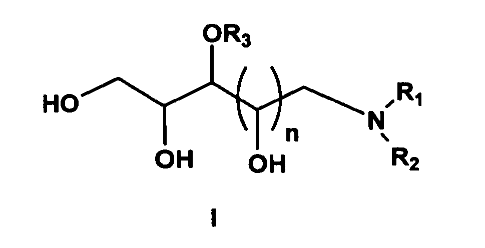 wherein n is an integer from 0 to 2;
and R₃ is selected from the group consisting of hydrogen, α-D-glucopyranosyl, β-D-glucopyranosyl, and β-D-galactopyranosyl and
a) R₁ is ethyl and R₂ is selected from the group consisting of n-pentyl, n-hexyl, and n-octyl, preferably n-hexyl; or
b) R₁ is n-butyl and R₂ is selected from the group consisting of n-butyl, n-pentyl, n-hexyl, and n-octyl, preferably n-butyl, n-pentyl, and n-hexyl.

40. The compound of claim 39, wherein most preferably R₁ is n-butyl and R₂ is n-pentyl.

## Patentansprüche

1. Verfahren zur Herstellung einer Verbindung gemäß Formel (I): 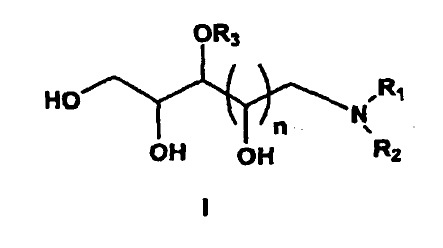 wobei n eine ganze Zahl von 0 bis 2 ist; R₁ und R₂ unabhängig ausgewählt sind aus der Gruppe bestehend aus C2 - C30 linearem Alkyl, cyclischem Alkyl, verzweigtem Alkyl, Alkenyl, Aryl, Aralkyl, Alkaryl, Alkoxyalkyl und Dialkylaminoalkyl; und R₃ ausgewählt ist aus der Gruppe bestehend aus Wasserstoff, α-D-Glucopyranosyl, β-D-Glucopyranosyl und β-D-Galactopyranosyl;
wobei das Verfahren das Inkontaktbringen eines N-Alkylpolyhydroxyalkylamins mit einer Carbonyläquivalentverbindung umfasst, ausgewählt aus der Gruppe bestehend aus Nitrilen, Ketonen, Aldehyden, Acetalen und Hemiacetalen, wobei das Inkontaktbringen in der Gegenwart von Wasserstoff und einem Übergangsmetallkatalysator in einem Reaktionslösungsmittel durchgeführt wird, umfassend mindestens 30 Gew.-% eines organischen Lösungsmittels, ausgewählt aus der Gruppe bestehend aus Methanol, Isopropanol, Tetrahydrofuran, Ethylenglykol, Propylenglykol, 1,2-Dimethoxyethan und Gemischen von diesen.

2. Verfahren nach Anspruch 1, wobei das organische Lösungsmittel mindestens 50 Gew.-% des Reaktionslösungsmittels, bevorzugt mindestens 70 Gew.-% des Reaktionslösungsmittels beiträgt.

3. Verfahren nach Anspruch 1, wobei das organische Lösungsmittel Methanol umfasst und bevorzugt Methanol ist und das Reaktionsgemisch wasserfrei ist.

4. Verfahren nach Anspruch 1, wobei die Carbonyläquivalentverbindung ein Aldehyd ist.

5. Verfahren nach Anspruch 1, wobei n 2 ist und R₃ H ist.

6. Verfahren nach Anspruch 7, wobei R₁ Ethyl ist und R₂ ausgewählt ist aus der Gruppe bestehend aus n-Pentyl, n-Hexyl und n-Octyl, bevorzugt n-Hexyl; oder R₁ n-Butyl ist und R₂ ausgewählt ist aus der Gruppe bestehend aus n-Butyl, n-Pentyl, n-Hexyl und n-Octyl, bevorzugt n-Butyl, n-Pentyl und n-Hexyl; und wobei am stärksten bevorzugt R₁ n-Butyl ist und R₂ n-Pentyl ist.

7. Verfahren nach Anspruch 6, wobei das Reaktionslösungsmittel mindestens 70 Gew.-% Methanol umfasst.

8. Verfahren nach Anspruch 1, wobei der Katalysator ausgewählt ist aus der Gruppe bestehend aus Eisen, Kobalt, Nickel, Ruthenium, Rhodium, Palladium, Osmium, Iridium und Platin, bevorzugt Ruthenium, Rhodium, Palladium und Platin, stärker bevorzugt Palladium oder Platin.

9. Verfahren nach Anspruch 1, wobei der Katalysator auf Kohlenstoff geträgert ist.

10. Verfahren nach Anspruch 1, wobei das Inkontaktbringen bei einer Temperatur zwischen etwa 50°C und etwa 175°C durchgeführt wird.

11. Formulierung, umfassend zwischen 0,1 und 99,9 Gew.-% insgesamt von einem oder mehreren Bestandteilen, ausgewählt aus der Gruppe bestehend aus grenzflächenaktiven Stoffen und Netzmitteln, welche verschieden von Formel (I) sind; Lösungsmitteln; Alkalimetallhydroxiden; Harzen auf Wasserbasis, wasserdispergierbaren oder wasserlöslichen Harzen; Fließmitteln; Egalisierhilfsmitteln; Pigmenten; Verarbeitungshilfsmitteln; Entschäumern; löslichmachenden Mitteln; Pestiziden; Pflanzenwachstummodifizierungsmitteln; wasserlöslichen filmbildenden Makromolekülen; wasserlöslichen Alkoholen, Glykolen oder Polyolen; wasserlöslichen Säuren oder Salzen davon; Tetramethylammoniumhydroxid; Emulgiermitteln; Alkanolaminen; organischen Monosäuren; Bioziden; Chelantmitteln; Detergenzaufbaustoffen; Detergenzcoaufbaustoffen; Farbstoffen; Duftstoffen; Hilfsmitteln gegen Wiederabscheidung; Sonnenschutzmitteln; löslichmachenden Mitteln; Polymeren; Oligomeren; funktionellen Zementadditiven; Natriumchlorid; Natriumbromid; Calciumchlorid; Calciumbromid; Zinkchlorid; Zinkbromid; Cäsiumformiat; Salzsäure; Fluorwasserstoffsäure; Essigsäure und Ameisensäure;
zwischen 0,001 und 45 Gew.-% von einer oder mehreren Verbindungen gemäß Formel (I): 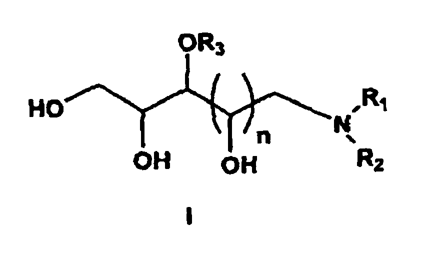 wobei n eine ganze Zahl von 0 bis 2 ist; R₁ und R₂ unabhängig ausgewählt sind aus der Gruppe bestehend aus C2 - C30 linearem Alkyl, cyclischem Alkyl, verzweigtem Alkyl, Alkenyl, Aryl, Aralkyl, Alkaryl, Alkoxyalkyl und Dialkylaminoalkyl; und R₃ ausgewählt ist aus der Gruppe bestehend aus Wasserstoff, α-D-Glucopyranosyl, β-D-Glucopyranosyl und β-D-Galactopyranosyl, wie in einem der Ansprüche 1 bis 10 definiert; und dem Rest Wasser zu 100 %;
wobei der eine oder mehrere Bestandteile keine Komponente eines Vor- oder Nachherstellung-Synthesereaktionsgemisches zur Herstellung von jedweder der einen oder mehreren Verbindungen gemäß Formel (I) einschließt.

12. Formulierung nach Anspruch 11, wobei die Formulierung eine Reinigungsformulierung für harte Oberflächen ist, umfassend Wasser und zwischen 0,1 und 99 Gew.-% insgesamt von einem oder mehreren Bestandteilen, ausgewählt aus der Gruppe bestehend aus anionischen grenzflächenaktiven Stoffen, kationischen grenzflächenaktiven Stoffen, nicht-ionischen grenzflächenaktiven Stoffen, welche verschieden von Formel (I) sind, Lösungsmitteln und Alkalimetallhydroxiden, wobei die Formulierung zwischen 0,001 und 25 Gew.-% von einer oder mehreren Verbindungen der Formel (I) umfasst.

13. Formulierung nach Anspruch 11, wobei die Formulierung eine Überzugsformulierung ist, umfassend zwischen 5 und 99,9 Gew.-% von einem Harz auf Wasserbasis, wasserdispergierbarem oder wasserlöslichem Harz und zwischen 0,01 und 10 Gew.-% insgesamt von einem oder mehreren anderen Additiven, ausgewählt aus der Gruppe bestehend aus grenzflächenaktiven Stoffen, Netzmitteln und Fließ- und Egalisierhilfsmitteln, welche verschieden von der Formel (I) sind, wobei die Formulierung zwischen 0,001 und 5 Gew.-% von einer oder mehreren Verbindungen der Formel (I) umfasst.

14. Formulierung nach Anspruch 11, wobei die Formulierung eine Tintenformulierung ist, umfassend zwischen 1 und 50 Gew.-% von einem Pigment, zwischen 5 und 99,9 Gew.-% von einem Harz auf Wasserbasis, wasserdispergierbarem oder wasserlöslichem Harz, zwischen 0,01 und 10 Gew.-% von einem grenzflächenaktiven Stoff oder Netzmittel, welche verschieden von Formel (I) sind, und zwischen 0,01 und 10 Gew.-% insgesamt von einem oder mehreren anderen Additiven, ausgewählt aus der Gruppe bestehend aus Verarbeitungshilfsmitteln, Entschäumern und löslichmachenden Mitteln, wobei die Formulierung zwischen 0,001 und 5 Gew.-% von einer oder mehreren Verbindungen der Formel (I) umfasst.

15. Formulierung nach Anspruch 11, wobei die Formulierung eine Formulierung für die Landwirtschaft ist, umfassend zwischen 0,1 und 50 Gew.-% von einem Pestizid oder Pflanzenwachstummodifizierungsmittel und zwischen 0,01 und 10 Gew.-% von einem grenzflächenaktiven Stoff oder Netzmittel, welche verschieden von Formel (I) sind, wobei die Formulierung zwischen 0,001 und 5 Gew.-% von einer oder mehreren Verbindungen der Formel (I) umfasst.

16. Formulierung nach Anspruch 11, wobei die Formulierung eine Feuchtmittelformulierung für Flachdruck ist, umfassend zwischen 0,05 und 10 Gew.-% von einem wasserlöslichen, filmbildenden Makromolekül, zwischen 1 und 25 Gew.-% von einem wasserlöslichen Alkohol, Glykol oder Polyol, zwischen 0,01 und 20 Gew.-% von einer wasserlöslichen Säure oder ihrem Salz und zwischen 30 und 98,9 Gew.-% Wasser, wobei die Formulierung zwischen 0,001 und 5 Gew.-% von einer oder mehreren Verbindungen der Formel (I) umfasst.

17. Formulierung nach Anspruch 11, wobei die Formulierung eine Photoresist-Entwicklerformulierung ist, umfassend zwischen 0,1 und 3 Gew.-% Tetramethylammoniumhydroxid und zwischen 92,5 und 99,9 Gew.-% Wasser, wobei die Formulierung zwischen 0,001 und 5 Gew.-% von einer oder mehreren Verbindungen der Formel (I) umfasst.

18. Formulierung nach Anspruch 11, wobei die Formulierung eine synthetische Metallbearbeitungsfluidformulierung ist, umfassend zwischen 2,5 und 10 Gew.-% von einem Emulgiermittel, zwischen 10 und 25 Gew.-% von einem Alkanolamin, zwischen 2 und 10 Gew.-% von einer organischen Monosäure, zwischen 1 und 5 Gew.-% von einem Biozid und zwischen 40 und 84,4 Gew.-% Wasser, wobei die Formulierung zwischen 0,001 und 5 Gew.-% von einer oder mehreren Verbindungen der Formel (I) umfasst.

19. Formulierung nach Anspruch 11, wobei die Formulierung eine Spülhilfsformulierung ist, umfassend Wasser und zwischen 5 und 20 Gew.-% von einem Chelantmittel, wobei die Formulierung zwischen 0,001 und 45 Gew.-% von einer oder mehreren Verbindungen der Formel (I) umfasst.

20. Formulierung nach Anspruch 11, wobei die Formulierung eine gepulverte Waschmittelformulierung ist, umfassend zwischen 0,1 und 50 Gew.-% von einem oder mehreren grenzflächenaktiven Detergenzien und zwischen 25 und 60 Gew.-% von einem Aufbaustoff oder Coaufbaustoff, wobei die Formulierung zwischen 0,001 und 15 Gew.-% von einer oder mehreren Verbindungen der Formel (I) umfasst.

21. Formulierung nach Anspruch 11, wobei die Formulierung eine wässrige Flüssigwaschmittelformulierung ist, umfassend zwischen 0,1 und 65 Gew.-% von einem oder mehreren grenzflächenaktiven Detergenzien, zwischen 3 und 36 Gew.-% von einem Aufbaustoff oder Coaufbaustoff, zwischen 0,1 und 5 Gew.-% insgesamt von einem oder mehreren anderen Additiven, ausgewählt aus der Gruppe, bestehend aus Duftstoffen und Farbstoffen, und zwischen 1 und 75 Gew.-% insgesamt von einem oder mehreren Additiven, ausgewählt aus der Gruppe bestehend aus Wasser und anderen Lösungsmitteln, wobei die Formulierung zwischen 0,001 und 30 Gew.-% von einer oder mehreren Verbindungen der Formel (I) umfasst.

22. Formulierung nach Anspruch 11, wobei die Formulierung eine nicht-wässrige Waschmittelformulierung ist, umfassend zwischen 0,1 und 42 Gew.-% von einem oder mehreren grenzflächenaktiven Detergenzien, zwischen 25 und 60 Gew.-% von einem Aufbaustoff oder Coaufbaustoff und zwischen 0,5 und 5 Gew.-% von einem Hilfsmittel gegen Wiederabscheidung, wobei die Formulierung zwischen 0,001 und 30 Gew.-% von einer oder mehreren Verbindungen der Formel (I) umfasst.

23. Formulierung nach Anspruch 11, wobei die Formulierung eine Industrie- und Anstaltswaschmittelformulierung ist, umfassend Wasser und zwischen 0,01 und 2 Gew.-% von einem Hilfsmittel gegen Wiederabscheidung, wobei die Formulierung zwischen 0,001 und 20 Gew.-% von einer oder mehreren Verbindungen der Formel (I) umfasst.

24. Formulierung nach Anspruch 11, wobei die Formulierung ein Shampoo- oder eine Körperwaschflüssigkeitsformulierung ist, umfassend Wasser und zwischen 0,1 und 30 Gew.-% von einem anionischen grenzflächenaktiven Stoff, wobei die Formulierung zwischen 0,001 und 5 Gew.-% von einer oder mehreren Verbindungen der Formel (I) umfasst.

25. Formulierung nach Anspruch 11, wobei die Formulierung eine HaarspülungsFormulierung ist, umfassend Wasser und zwischen 0,1 und 10 Gew.-% von einem nicht-ionischen grenzflächenaktiven Stoff, welcher verschieden von Formel (I) ist, wobei die Formulierung zwischen 0,001 und 10 Gew.-% von einer oder mehreren Verbindungen der Formel (I) umfasst.

26. Formulierung nach Anspruch 11, wobei die Formulierung eine wässrige Sonnenschutzformulierung ist, umfassend Wasser und zwischen 1 und 30 Gew.-% von einem Sonnenschutzmittel, wobei die Formulierung zwischen 0,001 und 30 Gew.-% von einer oder mehreren Verbindungen der Formel (I) umfasst.

27. Formulierung nach Anspruch 11, wobei die Formulierung eine Zementgemischformulierung ist, umfassend zwischen 40 und 75 Gew.-% Wasser und zwischen 0,1 und 20 Gew.-% insgesamt von einem oder mehreren löslichmachenden Mitteln, Polymeren, Oligomeren und funktionellen Additiven, wobei die Formulierung zwischen 0,001 und 5 Gew.-% von einer oder mehreren Verbindungen der Formel (I) umfasst.

28. Fluid zum Bohren, Komplettieren, Zementieren, Stimulieren, Brechen, Ansäuern oder Arbeiten über einer unterirdischen Gas- oder Ölquelle oder zum Behandeln oder Steigern der Produktion von Öl oder Gas aus einer öl- oder gasführenden Formation; wobei das Fluid zwischen 5 und 99,85 Gew.-% insgesamt von mindestens einem von einer organischen Flüssigkeit und Wasser umfasst und ferner zwischen 0,1 und 80 Gew.-% insgesamt von einem oder mehreren Bestandteilen, ausgewählt aus der Gruppe bestehend aus Streckmitteln, Viskositätsmitteln, Dispergiermitteln, Bohrschlamm-Ölgrundlagen, Emulgatoren, löslichen Salzen, Zementen, Stützmitteln, Mineralsäuren, organischen Säuren, Bioziden, Entschäumern, Demulgierem, Korrosionsinhibitoren, Reibungsverringungsmitteln, Gashydrat-Inhibitoren, Additiven zur Entfernung oder Steuerung von Schwefelwasserstoff, Additiven zur Steuerung von Asphalten, Additiven zur Steuerung von Paraffin und Additiven zur Steuerung von Kalkablagerungen;
und zwischen 0,05 und 10 Gew.-% von einer oder mehreren Verbindungen gemäß der Formel (I) umfasst: 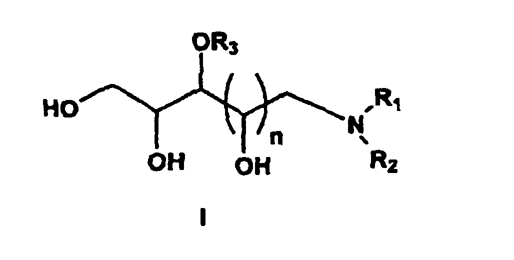 wobei n eine ganze Zahl von 0 bis 2 ist; R₁ und R₂ unabhängig ausgewählt sind aus der Gruppe bestehend aus C2 - C30 linearem Alkyl, cyclischem Alkyl, verzweigtem Alkyl, Alkenyl, Aryl, Aralkyl, Alkaryl, Alkoxyalkyl und Dialkylaminoalkyl; und R₃ ausgewählt ist aus der Gruppe bestehend aus Wasserstoff, α-D-Glucopyranosyl, β-D-Glucopyranosyl und β-D-Galactopyranosyl, wie in einem der Ansprüche 1 bis 10 definiert; und
wobei der eine oder mehrere Bestandteile keine Komponente eines Vor- oder Nachherstellung-Synthesereaktionsgemisches zur Herstellung von jedweder der einen oder mehreren Verbindungen gemäß Formel (I) einschließt.

29. Verfahren zum Bohren, Komplettieren, Zementieren, Stimulieren, Brechen, Ansäuern, Arbeiten über, oder Behandeln einer unterirdischen Quelle, wobei das Verfahren den Schritt des Einspritzens eines Fluids, umfasend eine oder mehreren Verbindungen gemäß Formel (I) in die Quelle einschließt: 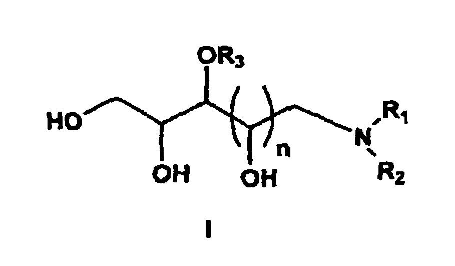 wobei n eine ganze Zahl von 0 bis 2 ist; R₁ und R₂ unabhängig ausgewählt sind aus der Gruppe bestehend aus C2 - C30 linearem Alkyl, cyclischem Alkyl, verzweigtem Alkyl, Alkenyl, Aryl, Aralkyl, Alkaryl, Alkoxyalkyl und Dialkylaminoalkyl; und R₃ ausgewählt ist aus der Gruppe bestehend aus Wasserstoff, α-D-Glucopyranosyl, β-D-Glucopyranosyl und β-D-Galactopyranosyl, wie in einem der Ansprüche 1 bis 10 definiert.

30. Verfahren nach Anspruch 29, wobei das Verfahren das Behandeln der Quelle zur Inhibierung von Gashydrat-Bildung umfasst.

31. Verfahren zum Behandeln eines hergestellten Öl- oder Gasstroms von einer öl- und gasführenden Formation, wobei das Verfahren den Schritt des Einspritzens eines Fluids umfassend eine oder mehr Verbindungen gemäß der Formel (I), in den hergestellten Strom umfasst: 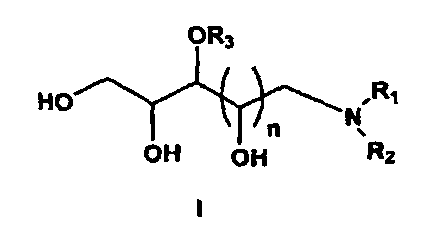 wobei n eine ganze Zahl von 0 bis 2 ist; R₁ und R₂ unabhängig ausgewählt sind aus der Gruppe bestehend aus C2 - C30 linearem Alkyl, cyclischem Alkyl, verzweigtem Alkyl, Alkenyl, Aryl, Aralkyl, Alkaryl, Alkoxyalkyl und Dialkylaminoalkyl; und R₃ ausgewählt ist aus der Gruppe bestehend aus Wasserstoff, α-D-Glucopyranosyl, β-D-Glucopyranosyl und β-D-Galactopyranosyl, wie in einem der Ansprüche 1 bis 10 definiert.

32. Verfahren nach Anspruch 31, wobei das Verfahren das Behandeln des hergestellten Stroms zur Inhibierung von Gashydrat-Bildung umfasst.

33. Formulierung, umfassend:
i) eine erste Komponente bestehend aus einer oder mehreren Verbindungen gemäß der Formel (I): 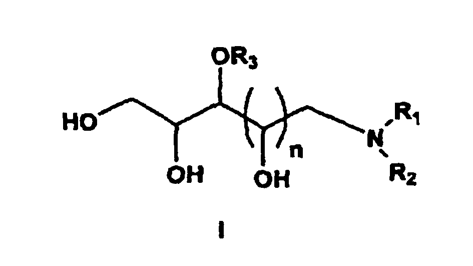 wobei n eine ganze Zahl von 0 bis 2 ist; R₁ und R₂ unabhängig ausgewählt sind aus der Gruppe bestehend aus C2 - C30 linearem Alkyl, cyclischem Alkyl, verzweigtem Alkyl, Alkenyl, Aryl, Aralkyl, Alkaryl, Alkoxyalkyl und Dialkylaminoalkyl; und R₃ ausgewählt ist aus der Gruppe bestehend aus Wasserstoff, α-D-Glucopyranosyl, β-D-Glucopyranosyl und β-D-Galactopyranosyl, wie in einem der Ansprüche 1 bis 10 definiert, und
ii) eine zweite Komponente bestehend aus einem oder mehreren Materialien, ausgewählt aus der Gruppe bestehend aus nichtflüchtigen organischen und nichtflüchtigen anorganischen Materialien und Gemischen davon, wobei die zweite Komponente keine Komponente eines Vor- oder Nachherstellung-Synthesereaktionsgemisches zur Herstellung von jedweder der einen oder mehreren Verbindungen gemäß Formel (I) einschließt;
wobei die Formulierung fluid bei 25°C ist.

34. Formulierung nach Anspruch 33, wobei die zweite Komponente in einer größeren Menge, bezogen auf das Gewicht, als die erste Komponente vorhanden ist.

35. Formulierung nach Anspruch 34, wobei die Formulierung einen wässrigen Träger umfasst.

36. Formulierung nach Anspruch 33, wobei die zweite Komponente aus einem oder mehreren Materialien besteht, ausgewählt aus der Gruppe bestehend aus grenzflächenaktiven Stoffen oder Netzmitteln, welche verschieden von Formel (I) sind; Lösungsmitteln; Alkalimetallhydroxiden; Harzen auf Wasserbasis, wasserdispergierbaren oder wasserlöslichen Harzen; Fließmitteln; Egalisierhilfsmitteln; Pigmenten; Verarbeitungshilfsmitteln; Entschäumern; löslichmachenden Mitteln; Pestiziden; Pflanzenwachstumsmodifizierungsmitteln; wasserlöslichen filmbildenden Makromolekülen; wasserlöslichen Alkoholen, Glykolen oder Polyolen; wasserlöslichen Säuren oder Salzen davon; Tetramethylammoniumhydroxid; Emulgatoren; Alkanolaminen; organischen Monosäuren; Bioziden; Chelantmitteln; Detergenzaufbaustoffen; Detergenzcoaufbaustoffen; Farbstoffen; Duftstoffen; Hilfsmitteln gegen Wiederabscheidung; Sonnenschutzmitteln; löslichmachenden Mitteln; Polymeren; Oligomeren und funktionellen Zementadditiven.

37. Formulierung nach Anspruch 36, wobei R₁ Ethyl ist und R₂ ausgewählt ist aus der Gruppe bestehend aus n-Pentyl, n-Hexyl und n-Octyl, bevorzugt n-Hexyl; oder R₁ n-Butyl ist und R₂ ausgewählt ist aus der Gruppe bestehend aus n-Butyl, n-Pentyl, n-Hexyl und n-Octyl, bevorzugt n-Butyl, n-Pentyl und n-Hexyl; und wobei am stärksten bevorzugt R₁ n-Butyl ist und R₂ n-Pentyl ist.

38. Verwendung einer Verbindung der Formel (I) wie in den Ansprüchen 1 bis 10 definiert in einer Formulierung, ausgewählt aus der Gruppe bestehend aus einer Reinigungsformulierung für harte Oberflächen, einer Überzugsformulierung, einer Tintenformulierung, einer Formulierung für die Landwirtschaft, einer Feuchtmittelformulierung, einer Photoresist-Entwicklerformulierung, einer synthetischen Metallbearbeitungsfluidformulierung, einer Spülhilfsmittelformulierung, einer gepulverten Waschmittelformulierung, einer wässrigen Flüssigwaschmittelformulierung, einer nichtwässrigen Waschmittelformulierung, einer Industrie- und Anstaltswaschmittelformulierung, einer Shampoo- oder Körperwaschflüssigkeitformulierung, einer Haarspülungsformulierung, einer wässrigen Sonnenschutzformulierung und einer Zementgemischformulierung.

39. Verbindung der Formel (I): 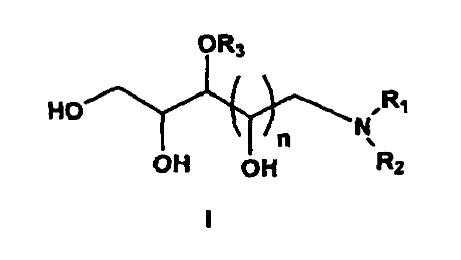 wobei n eine ganze Zahl von 0 bis 2 ist;
und R₃ ausgewählt ist aus der Gruppe bestehend aus Wasserstoff, α-D-Glucopyranosyl,
β-D-Glucopyranosyl und β-D-Galactopyranosyl, und
a) R₁ Ethyl ist und R₂ ausgewählt ist aus der Gruppe bestehend aus n-Pentyl, n-Hexyl und n-Octyl, bevorzugt n-Hexyl; oder
b) R₁ n-Butyl ist und R₂ ausgewählt ist aus der Gruppe bestehend aus n-Butyl, n-Pentyl, n-Hexyl und n-Octyl, bevorzugt n-Butyl, n-Pentyl und n-Hexyl.

40. Verbindung nach Anspruch 39, wobei am stärksten bevorzugt R₁ n-Butyl ist und R₂ n-Pentyl ist.

## Revendications

1. Procédé de fabrication d'un composé selon la formule (I) : 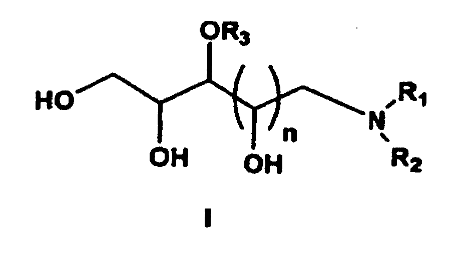 dans laquelle n est un entier de 0 à 2 ; R₁ et R₂ sont indépendamment choisis dans le groupe consistant en un alkyle linéaire en C₂-C₃₀, un alkyle cyclique, un alkyle ramifié, un alcényle, un aryle, un aralkyle, un alkaryle, un alkoxyalkyle et un dialkylaminoalkyle ; et R₃ est choisi dans le groupe consistant en hydrogène, un α-D-glucopyranosyle, un β-D-glucopyranosyle et β-D-galactopyranosyle ;
le procédé comprenant la mise en contact d'un N-alkylpolyhydroxyalkylamine avec un composant équivalent au carbonyle choisi dans le groupe consistant en des nitriles, des cétones, des aldéhydes, des acétals et des hémi-acétals, ledit contact étant effectué en présence d'hydrogène et d'un catalyseur de métal de transition dans un solvant de réaction comprenant au moins 30 % en poids d'un solvant organique choisi dans le groupe consistant en du méthanol, de l'isopropanol, du tétrahydrofurane, de l'éthylène glycol, du propylène glycol, du 1,2-diméthoxyéthane et des mélanges de ceux-ci.

2. Procédé selon la revendication 1, dans lequel le solvant organique constitue au moins 50 % en poids du solvant de réaction, de préférence, au moins 70 % en poids du solvant de réaction.

3. Procédé selon la revendication 1, dans lequel le solvant organique comprend le méthanol et est, de préférence, du méthanol et le mélange de réaction est anhydre.

4. Procédé selon la revendication 1, dans lequel le composé équivalent au carbonyle est un aldéhyde.

5. Procédé selon la revendication 1, dans lequel n est 2 et R₃ est H.

6. Procédé selon la revendication 7, dans lequel R₁ est éthyle et R₂ est choisi dans le groupe consistant en du n-pentyle, du n-hexyle et de n-octyle, de préférence, du n-hexyle ; ou R₁ est n-butyle et R₂ est choisi dans le groupe consistant en du n-butyle, du n-pentyle, du n-hexyle et du n-octyle, de préférence, n-butyle, n-pentyle et n-hexyle ; et dans lequel, de façon préférée entre toutes, R₁ est n-butyle et R₂ est n-pentyle.

7. Procédé selon la revendication 6, dans lequel le solvant de réaction comprend au moins 70 % en poids de méthanol.

8. Procédé selon la revendication 1, dans lequel le catalyseur est choisi dans le groupe consistant en du fer, du colbalt, du nickel, du ruthénium, du rhodium, du palladium, de l'osmium, de l'iridium et du platine, de préférence, du ruthénium, du rhodium, du palladium et du platine, de préférence encore, du palladium ou du platine.

9. Procédé selon la revendication 1, dans lequel le catalyseur est supporté sur du carbone.

10. Procédé selon la revendication 1, dans lequel le contact est effectué à une température entre environ 50 °C et environ 175 °C.

11. Formulation comprenant entre 0,1 et 99,9 % en poids au total d'un ou plusieurs ingrédients choisis dans le groupe consistant en des tensioactifs et des agents mouillants autres que selon la formule (I) ; des solvants ; des hydroxydes de métal alcalin ; des résines à base d'eau, hydrodispersables ou hydrosolubles ; des agents fluidifiants ; des agents d'étalement ; des pigments ; des adjuvants de fabrication ; des agents antimousse ; des agents de solubilisation ; des pesticides ; des agents modifiant la croissance des plantes; des macromolécules de formation de film hydrosolubles ; des alcools, glycols ou polyols hydrosolubles ; des acides hydrosolubles ou sels de ceux-ci ; de l'hydroxyde de tétraméthylammonium ; des agents émulsifiants ; des alkanolamines ; des monoacides organiques ; des biocides ; des agents chélateurs ; des adjuvants de détersifs; des coadjuvants de détersifs ; des colorants ; des parfums ; des agents anti-redéposition ; des agents écrans solaires ; des agent de solubilisation ; des polymères ; des oligomères ; des additifs à ciment fonctionnels ; du chlorure de sodium ; du bromure de sodium ; du chlorure de calcium ; du bromure de calcium ; du chlorure de zinc ; du bromure de zinc ; du formate de césium ; de l'acide chlorhydrique ; de l'acide acétique et de l'acide formique ;
entre 0,001 et 45 % en poids d'un ou plusieurs composés selon la formule (I) : 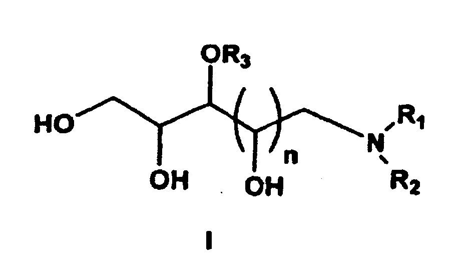 dans laquelle n est un entier de 0 à 2 ; R₁ et R₂ sont choisis indépendamment dans le groupe consistant en un alkyle linéaire en C₂ - C₃₀, un alkyle cyclique, un alkyle ramifié, un alcényle, un aryle, un aralkyle, un alkaryle, un alcoxyalkyle et un dialkylaminoalkyle ; et R₃ est choisi dans le groupe consistant en l'hydrogène, un α-D-glucopyranosyle, un β-D-glucopyranosyle et un β-D-galactopyranosyle, comme défini dans une des revendications 1 à 10 ; et le complément de l'eau jusqu'à 100 % en poids ;
où lesdits un ou plusieurs ingrédients n'incluent pas un quelconque composant d'un mélange de réaction de synthèse pré- ou post-préparation pour la préparation d'un quelconque des un ou plusieurs composés selon la formule (I).

12. Formulation selon la revendication 11, dans laquelle la formulation est une formulation de nettoyage de surface dure comprenant de l'eau et entre 0,1 et 99 % en poids au total d'un ou plusieurs ingrédients choisis dans le groupe consistant en des tensioactifs anioniques, des tensioactifs cationiques, des tensioactifs non ioniques autres que selon la formule (I), des solvants et des hydroxydes de métal alcalin, la formulation comprenant entre 0,001 et 25 % en poids d'un ou plusieurs composés de formule (I).

13. Formulation selon la revendication 11, dans laquelle la formulation est une formulation de revêtement comprenant entre 5 et 99.9 % en poids d'une résine à base d'eau, hydrodispersable ou hydrosoluble, et entre 0.01 et 10 % en poids au total d'un ou plusieurs autres additifs choisis dans le groupe consistant en des tensioactifs, des agents mouillants et des agents fluidifiants et d'étalement, autres que selon la formule (I), la formulation comprenant entre 0,001 et 5 % en poids d'un ou plusieurs composés de formule (I).

14. Formulation selon la revendication 11, dans laquelle la formulation est une formulation d'encre comprenant entre 1 et 50 % en poids d'un pigment, entre 5 et 99,9 % en poids d'une résine à base d'eau, hydrodispersable ou hydrosoluble, entre 0,01 et 10 % en poids d'un tensioactif ou agent mouillant autre que selon la formule (I), et entre 0.01 et 10 % en poids au total d'un ou plusieurs autres additifs choisis dans le groupe consistant en des adjuvants de fabrication, des agents antimousse et des agents de solubilisation, la formulation comprenant entre 0,001 et 5 % en poids d'un ou plusieurs composés de formule (1).

15. Formulation selon la revendication 11, dans laquelle la formulation est une formulation agricole comprenant entre 0,1 et 50 % en poids d'un pesticide ou d'un agent modifiant la croissance des plantes et entre 0,01 et 10 % en poids d'un tensioactif ou agent mouillant autre que selon la formule (I), la formulation comprenant entre 0,001 et 5 % en poids d'un ou plusieurs composés de formule (I).

16. Formulation selon la revendication 11, dans laquelle la formulation est une formulation de solution de mouillage pour impression planographique comprenant entre 0,05 et 10 % en poids d'une macromolécule de formation de film hydrosoluble, entre 1 et 25 % en poids d'un alcool, glycol ou polyol hydrosoluble, entre 0,01 et 20 % en poids d'un acide hydrosoluble ou de son sel, et entre 30 et 98,9 % en poids d'eau, la formulation comprenant entre 0,001 et 5 % en poids d'un ou plusieurs composés de formule (I).

17. Formulation selon la revendication 11, dans laquelle la formulation est une formulation de révélateur photorésistant comprenant entre 0,1 et 3 % en poids d'hydroxyde de tétraméthylammonium et entre 92,5 et 99,9 % en poids d'eau, la formulation comprenant entre 0,001 et 5 % en poids d'un ou plusieurs composés de formule (I).

18. Formulation selon la revendication 11, dans laquelle la formulation est une formulation de fluide de travail de métal synthétique comprenant entre 2,5 et 10 % en poids d'un agent émulsifiant, entre 10 et 25 % en poids d'une alkanolamine, entre 2 et 10 % en poids d'un monoacide organique, entre 1 et 5 % en poids d'un biocide, et entre 40 et 84,4 % en poids d'eau, la formulation comprenant entre 0,001 et 5 % en poids d'un ou plusieurs composés de formule (I).

19. Formulation selon la revendication 11, dans laquelle la formulation est une formulation d'aide au rinçage comprenant de l'eau et entre 5 et 20 % en poids d'un agent chélateur, la formulation comprenant entre 0,001 et 45 % en poids d'un ou plusieurs composés de formule (I).

20. Formulation selon la revendication 11, dans laquelle la formulation est une formulation de détergent à lessive en poudre comprenant entre 0,1 et 50 % en poids d'un ou plusieurs tensioactifs détergents et entre 25 et 60 % en poids d'un adjuvant or coadjuvant, la formulation comprenant entre 0,001 et 15 % en poids d'un ou plusieurs composés de formule (I).

21. Formulation selon la revendication 11, dans laquelle la formulation est une formulation de détergent à lessive liquide aqueuse comprenant entre 0,1 et 65 % en poids d'un ou plusieurs tensioactifs détergents, entre 3 et 36 % en poids d'un adjuvant ou coadjuvant, entre 0,1 et 5 % en poids au total d'un ou plusieurs autres additifs choisis dans le groupe consistant en des parfums et des colorants, et entre 1 et 75 % en poids au total d'un ou plusieurs autres additifs choisis dans le groupe consistant en de l'eau et d'autres solvants, la formulation comprenant entre 0,001 et 30 % en poids d'un ou plusieurs composés de formule (I).

22. Formulation selon la revendication 11, dans laquelle la formulation est une formulation de détergent à lessive non aqueuse comprenant entre 0,1 et 42 % en poids d'un ou plusieurs tensioactifs détergents, entre 25 et 60 % en poids d'un adjuvant ou coadjuvant et entre 0,5 et 5 % en poids d'un agent anti-redéposition, la formulation comprenant entre 0,001 et 30 % en poids d'un ou plusieurs composés de formule (I).

23. Formulation selon la revendication 11, dans laquelle la formulation est une formulation de détergent à lessive industriel et d'établissement comprenant de l'eau et entre 0,01 et 2 % en poids d'un agent anti-redéposition, la formulation comprenant entre 0,001 et 20 % en poids d'un ou plusieurs composés de formule (I).

24. Formulation selon la revendication 11, dans laquelle la formulation est une formulation de shampooing ou de savon liquide pour le corps comprenant de l'eau et entre 0,1 et 30 % en poids d'un tensioactif anionique, la formulation comprenant entre 0,001 et 5 % en poids d'un ou plusieurs composés de formule (I).

25. Formulation selon la revendication 11, dans laquelle la formulation est une formulation d'après-shampooing comprenant de l'eau et entre 0,1 et 10 % en poids d'un tensioactif non ionique autre que selon la formule (I), la formulation comprenant entre 0,001 et 10 % en poids d'un ou plusieurs composés de formule (I).

26. Formulation selon la revendication 11, dans laquelle la formulation est une formulation d'écran solaire aqueuse comprenant de l'eau et entre 1 et 30 % en poids d'un agent écran solaire, la formulation comprenant entre 0,001 et 30 % en poids d'un ou plusieurs composés de formule (I).

27. Formulation selon la revendication 11, dans laquelle la formulation est une formulation d'adjuvant de ciment comprenant entre 40 et 75 % en poids d'eau et entre 0,1 et 20 % en poids au total d'un ou plusieurs agents de solubilisation, polymères, oligomères ou additifs fonctionnels, la formulation comprenant entre 0,001 et 5 % en poids d'un ou plusieurs composés de formule (I).

28. Fluide pour forer, finir, cimenter, stimuler, fracturer, acidifier ou travailler sur un puits de gaz ou de pétrole souterrain, ou pour traiter ou améliorer la production de pétrole ou de gaz à partir d'une formation pétrolifère ou gazéifère ; le fluide comprenant entre 5 et 99,85 % en poids au total d'au moins un d'un liquide organique et de l'eau et comprenant en outre entre 0,1 et 80 % en poids au total d'un ou plusieurs ingrédients choisis dans le groupe consistant en des agents alourdissants, des agents améliorant la viscosité, des dispersants, des huiles à base de boue de forage, des émulsifiants, des sels solubles, des ciments, des agents de soutènement, des acides minéraux, des acides organiques, des biocides, des agents antimousse, des désémulsifiants, des inhibiteurs de corrosion, des réducteurs de frottement, des inhibiteurs d'hydrate de gaz, des additifs d'élimination ou de contrôle de sulfure d'hydrogène, des additifs de contrôle d'asphaltène, des additifs de contrôle de paraffine et des additifs de contrôle de calamine ;
et entre 0,05 et 10 % en poids d'un ou plusieurs composés selon la formule (I) : 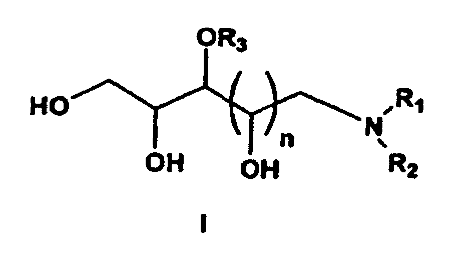 dans laquelle n est un entier de 0 à 2 ; R₁ et R₂ sont indépendamment choisis dans le groupe consistant en un alkyle linéaire en C₂ - C₃₀, un alkyle cyclique, un alkyle ramifié, un alcényle, un aryle, un aralkyle, un alcoxyalkyle et un dialkylaminoalkyle ; et R₃ est choisi dans le groupe consistant en l'hydrogène, un α-D-glucopyranosyle, un β-D-glucopyranosyle et un β-D-galactopyranosyle ; comme défini dans une des revendications 1 à 10 ; et
où lesdits un ou plusieurs ingrédients n'incluent pas un quelconque composant d'un mélange de réaction de synthèse pré- ou post-préparation pour la préparation de l'un quelconque des un ou plusieurs composés selon la formule (I).

29. Procédé pour forer, finir, cimenter, stimuler, fracturer, acidifier, travailler sur ou traiter un puits souterrain, ledit procédé comprenant l'étape consistant à injecter dans le puits un fluide comprenant un ou plusieurs composés selon la formule (I) : 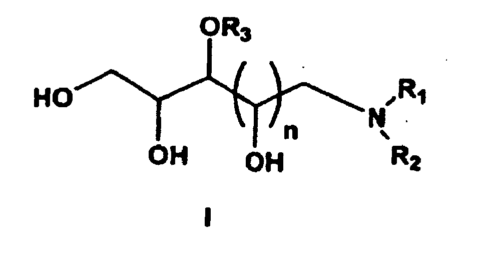 dans laquelle n est un entier de 0 à 2 ; R₁ et R₂ sont indépendamment choisis dans le groupe consistant en un alkyle linéaire en C₂ - C₃₀, un alkyle cyclique, un alkyle ramifié, un alcényle, un aryle, un aralkyle, un alkaryle, un alcoxyalkyle et un dialkylaminoalkyle ; et R₃ est choisi dans le groupe consistant en l'hydrogène, un α-D-glucopyranosyle, un β-D-glucopyranosyle et un β-D-galactopyranosyle, comme défini selon l'une des revendications 1 à 10.

30. Procédé selon la revendication 29, dans lequel le procédé consiste à traiter le puits pour inhiber la formation d'hydrate de gaz.

31. Procédé destiné à traiter un flux d'huile ou de gaz produit à partir d'une formation pétrolifère ou gazéifère, ledit procédé comprenant l'étape consistant à injecter dans le flux produit un fluide comprenant un ou plusieurs composés selon la formule (I) : 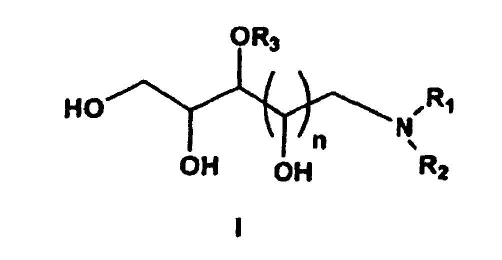 dans laquelle n est un entier de 0 à 2 ; R₁ et R₂ sont indépendamment choisis dans le groupe consistant en un alkyle linéaire en C₂ - C₃₀, un alkyle cyclique, un alkyle ramifié, un alcényle, un aryle, un aralkyle, un alkaryle, un alcoxyalkyle et un dialkylaminoalkyle ; et R₃ est choisi dans le groupe consistant en l'hydrogène, un α-D-glucopyranosyle, un β-D-glucopyranosyle et un β-D-galactopyranosyle, comme défini selon l'une des revendications 1 à 10.

32. Procédé selon la revendication 31, dans lequel le procédé consiste à traiter le flux produit pour inhiber la formation d'hydrate de gaz.

33. Formulation comprenant :
i) un premier composant comprenant un ou plusieurs composés selon la formule (I) : 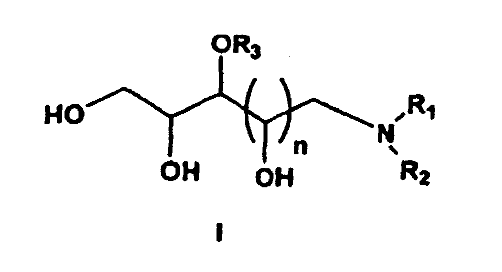 dans laquelle n est un entier de 0 à 2 ; R₁ et R₂ sont indépendamment choisis dans le groupe consistant en un alkyle linéaire en C₂ - C₃₀, un alkyle cyclique, un alkyle ramifié, un alcényle, un aryle, un aralkyle, un alkaryle, un alkoxyalkyle et un dialkylaminoalkyle ; et R₃ est choisi dans le groupe consistant en l'hydrogène, un α-D-glucopyranosyle, un β-D-glucopyranosyle et un β-D-galactopyranosyl; comme défini selon l'une des revendications 1 à 10 et
ii) un second composant consistant en une ou plusieurs matières choisies dans le groupe consistant en des matières organiques non volatiles et inorganiques non volatiles et des mélanges de celles-ci, ledit second composant ne comprenant pas un quelconque composant d'un mélange de réaction de synthèse pré- ou post-préparation pour la préparation de l'un quelconque des un ou plusieurs composés selon la formule (I) ;
dans laquelle la formulation est fluide à 25 °C.

34. Formulation selon la revendication 33, dans laquelle le second composant est présent dans une plus grande quantité en poids que le premier composant.

35. Formulation selon la revendication 34, dans laquelle la formulation comprend un véhicule aqueux.

36. Formulation selon la revendication 33, dans laquelle le second composant comprend une ou plusieurs matières choisies dans le groupe consistant en des tensioactifs ou des agents mouillants autres que selon la formule (I) ; des solvants ; hydroxydes de métal alcalin ; des résines à base d'eau, des hydrodispersables ou des hydrosolubles ; des agents fluidifiants ; des agents d'étalement ; pigments ; des adjuvants de fabrication ; des agents antimousse ; des agents de solubilisation ; des pesticides ; des agents modifiant la croissance des plantes : des macromolécules de formation de film hydrosolubles ; des alcools, des glycols ou des polyols hydrosolubles ; des acides hydrosolubles ou ses els de ceux-ci ; de l'hydroxyde de tétraméthylammonium ; des agents émulsifiants ; des alkanolamines ; monoacides organiques ; des biocides ; des agents chélateurs ; des adjuvants détergents, des coadjuvants détergents ; des colorants ; des parfums ; des agents anti-redéposition ; des agents écrans solaires ; des agents de solubilisation ; des polymères ; des oligomères et des additifs de ciment fonctionnels.

37. Formulation selon la revendication 36, dans laquelle R₁ est éthyle et R₂ est choisi dans le groupe consistant en du n-pentyle, du n-hexyle et du n-octyle, de préférence, du n-hexyle ; ou R₁ est du n-butyle et R₂ est choisi dans le groupe consistant en du n-butyle, du n-pentyle, du n-hexyle et du n-octyle, de préférence, du n-butyle, du n-pentyle et du n-hexyle ; et dans laquelle, de façon préférée entre toutes, R₁ est n-butyle et R₂ est n-pentyle.

38. Utilisation d'un composé de formule (I) selon une des revendications 1 à 10 dans une formulation choisie dans le groupe consistant en une formulation de nettoyage de surface dure, une formulation de revêtement, une formulation d'encre, une formulation agricole, une formulation de solution de mouillage, une formulation de révélateur photorésistant, une formulation de fluide de travail de métal synthétique, une formulation d'aide au rinçage, une formulation de détergent à lessive en poudre, une formulation de détergent à lessive liquide aqueuse, une formulation de détergent à lessive non-aqueuse, une formulation de détergent à lessive industrielle et d'établissement, une formulation de shampooing ou de savon liquide pour le corps, une formulation d'après-shampooing, une formulation d'écran solaire aqueuse et une formulation d'additif à ciment.

39. Composé de formule (I) : 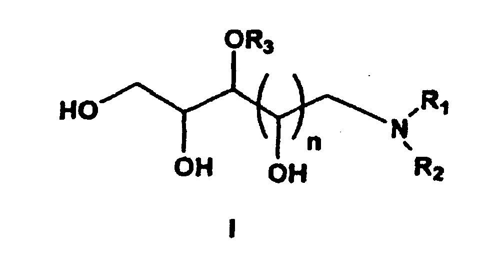 dans laquelle n est un entier de 0 à 2 ;
et R₃ est choisi dans le groupe consistant l'hydrogène, un α-D-glucopyranosyle, un β-D-glucopyranosyle et un β-D-galactopyranosyl, et
a) R₁ est éthyle et R₂ est choisi dans le groupe consistant en du n-pentyle, du n-hexyle et du n-octyle, de préférence, du n-hexyle ; ou
b) R₁ est n-butyle et R₂ est choisi dans le groupe consistant en du n-butyle, du n-pentyle, du n-hexyle et du n-octyle, de préférence, du n-butyle, du n-pentyle et du n-hexyle.

40. Composé selon la revendication 39, dans lequel, de façon préférée entre toutes, R₁ est n-butyle et R₂ est n-pentyle.
